# EUROPEAN PATENT SPECIFICATION

(11) **EP 3 104 884 B1**
(45) Date of publication and mention of the grant of the patent: **13.05.2020**
(21) Application number: 15707819.7
(22) Date of filing: 05.02.2015
(51) Int. Cl.: A61K 39/02, C07K 16/12, G01N 33/48, A61P 31/18

(54) **A NEW NON-HIV VACCINE ANTIGEN FROM THE VAGINAL MICROBIOTA CAPABLE OF INDUCING A MUCOSAL NEUTRALIZING PROTECTIVE ANTIBODY RESPONSE AGAINST HIV INFECTION**
NEUES, NICHT-HIV-IMPFSTOFF-ANTIGEN AUS DER VAGINALEN MIKROBIOTA ZUR INDUZIERUNG EINER MUKOSALEN, NEUTRALISIERENDEN, SCHÜTZENDEN ANTIKÖRPERREAKTION GEGEN EINE HIV-INFEKTION
NOUVEL ANTIGÈNE VACCINAL NON-VIH ISSU DU MICROBIOTE VAGINAL, APTE À INDUIRE UNE RÉPONSE D'ANTICORPS PROTECTEURS NEUTRALISANTS MUQUEUX CONTRE L'INFECTION À VIH

(30) Priority: 10.02.2014 EP 14305174
(43) Date of publication of application: 21.12.2016
(73) Proprietor: B Cell Design, 87000 Limoges (FR)
(72) Inventor: EL HABIB, Raphaëlle Claude, F-69630 Chaponost (FR); SODOYER, Régis, F-69290 Saint Genis Les Ollieres (FR); CUVILLIER, Armelle, F-87510 Saint-Jouvent (FR); MOOG, Christiane, F-67540 Ostwald (FR)
(74) Representative: Plasseraud IP
(86) International application number: PCT/IB2015/050870
(87) International publication number: WO 2015/118473

(56) References cited:
- WO-A1-2012/066331
- VUJANOVIC LAZAR ET AL: "A mycoplasma peptide elicits heteroclitic CD4+ T cell responses against tumor antigen MAGE-A6.", CLINICAL CANCER RESEARCH : AN OFFICIAL JOURNAL OF THE AMERICAN ASSOCIATION FOR CANCER RESEARCH 15 NOV 2007, vol. 13, no. 22 Pt 1, 15 November 2007 (2007-11-15), pages 6796-6806, XP002726235, ISSN: 1078-0432
- FRASER C M ET AL: "THE MINIMAL GENE COMPLEMENT OF MYCOPLASMA GENITALIUM", SCIENCE, AMERICAN ASSOCIATION FOR THE ADVANCEMENT OF SCIENCE, US, vol. 270, no. 5235, 20 October 1995 (1995-10-20), pages 397-403, XP000942028, ISSN: 0036-8075, DOI: 10.1126/SCIENCE.270.5235.397 -& DATABASE UniProt [Online] 1 November 1997 (1997-11-01), "RecName: Full=Uncharacterized ABC transporter permease MG468;", XP002726236, retrieved from EBI accession no. UNIPROT:Q49460 Database accession no. Q49460
- AZEEM MEHMOOD BUTT ET AL: ": A comparative genomics study of metabolic pathways for the identification of drug and vaccine targets", INFECTION, GENETICS AND EVOLUTION, ELSEVIER, AMSTERDAM, NL, vol. 12, no. 1, 10 October 2011 (2011-10-10), pages 53-62, XP028340909, ISSN: 1567-1348, DOI: 10.1016/J.MEEGID.2011.10.017 [retrieved on 2011-10-25]
- CATHERINE L. HAGGERTY ET AL: "Mycoplasma genitalium: An Emerging Cause of Pelvic Inflammatory Disease", INFECTIOUS DISEASES IN OBSTETRICS & GYNECOLOGY, vol. 47, no. 12, 1 January 2011 (2011-01-01), pages 1115-9, XP055125073, ISSN: 1064-7449, DOI: 10.1111/j.1471-0528.2010.02687.x
- CLYDE A HUTCHISON ET AL: "Global Transposon Mutagenesis and a Mineral Mycoplasma Genome", SCI, AMERICAN ASSOCIATION FOR THE ADVANCEMENT OF SCIENCE, vol. 286, 10 December 1999 (1999-12-10), pages 2165-2169, XP008128717, ISSN: 0036-8075, DOI: 10.1126/SCIENCE.286.5447.2165
- GLASS JOHN I ET AL: "Essential genes of a minimal bacterium", 20060101; 20060100, vol. 103, no. 2, 1 January 2006 (2006-01-01), pages 425-430, XP002419929,

### SEQUENCE LISTING

<110> B CELL DESIGN
<120> A NEW NON-HIV VACCINE ANTIGEN FROM THE VAGINAL MICROBIOTA CAPABLE OF INDUCING A MUCOSAL NEUTRALIZING PROTECTIVE ANTIBODY RESPONSE AGAINST HIV INFECTION
<130> 2498PCT2
<160> 20
<170> PatentIn version 3.5
<210> 1
   <211> 1783
   <212> PRT
   <213> Mycoplasma genitalium
<400> 1
   Met Phe Ser Phe Phe Lys Gln Ile Phe Lys Ser Leu Lys Lys Phe Phe
   1 5 10 15
   Phe Leu Leu Phe Gly Ile Ile Phe Val Leu Phe Ser Ile Ile Phe Leu
   20 25 30
   Glu Thr Ser Ile Val Gln Leu Ser Asn Asn Leu Val Ser Thr Tyr Thr
   35 40 45
   Thr Leu Val Ser Lys Thr Asn Ser Ser Asp Ile Val Ala Pro Ala Ile
   50 55 60
   Leu Lys Glu Ala Asn Pro Val Tyr Ile Ala Ser Leu Thr Asn Asp Ser
   65 70 75 80
   Gly Tyr Phe Ser Lys Ile Lys Ile Asp Asp Lys Lys Ile Asn Tyr Leu
   85 90 95
   Phe Pro Tyr Gln Glu Asn Asp Phe Gly Ser Asp Ser Gly Gln Ser Asn
   100 105 110
   Gly Ser Gly Asp Asn Gln Asn Lys Thr Ile Pro Arg Lys Gly Asp Val
   115 120 125
   Asn Glu Lys Asp Lys Leu Phe Leu Ala Arg Lys Arg Gly Ile Leu Lys
   130 135 140
   Ala Tyr Gly Glu Ala Asn Ile Ala Glu Lys Arg Ile Tyr Lys Gly Leu
   145 150 155 160
   Ala Val Ser Phe Asn Asn Thr Asp Ser Phe Asn Gly Ser Asp Ile Ser
   165 170 175
   Asp Ser Ile Thr Asn Arg His Ile Ile Ser Asp Pro Gln Asn Leu Ile
               180                 185                 190
   Tyr Asp Ala Ser Gly Asn Leu Leu Gly Tyr Phe Ala Asp Gly Leu Ile
           195                 200                 205
   Lys Glu Thr Ile Ser Leu Arg Ala Gly Ile Ala Arg Phe Pro Gly Asp
       210                 215                 220
   Lys Gly Lys Ser Thr Gly Thr Gln Val Lys Ile Thr Gln Lys Gln Gln
   225                 230                 235                 240
   Thr Asn Asn Asp Pro Gln Lys Asp Ser Thr Val Asn Ser Leu Tyr Lys
                   245                 250                 255
   Thr Asn Asn Lys Asp Lys Val Trp Phe Lys Ser Asp Glu Thr Lys Ala
               260                 265                 270
   Asp Asn Thr Asp Ile Ser Ala Asn Tyr Leu Phe Thr Gly Gly Asn Glu
           275                 280                 285
   Ala Ala Asn Trp Phe Pro Asn Leu Tyr Ala Asn Ile Pro Ile Asp Leu
       290                 295                 300
   Glu Ile Asp Pro Gly Ser Gln Phe Trp Lys Asp Val Asn Pro Phe Lys
   305                 310                 315                 320
   Glu Ile Val Glu Glu Phe Gln Thr Gln Lys Glu Ser Lys Asp Asn Gln
                   325                 330                 335
   Ser Phe Thr Leu Thr Phe Asn Leu Asp Ile Ser Lys Leu Asn Lys Leu
               340                 345                 350
   Asp Asn Glu Gln Leu Lys Trp Leu Glu Thr Asn Ala Lys Thr Ile Ala
           355                 360                 365
   Asn Asn Ser Ser Phe Gly Asp Trp Asp Leu Glu Asn Lys Leu Lys Gln
       370                 375                 380
   Leu Lys Lys Phe Glu Leu Lys Ile Asn Lys Asp Trp Leu Lys Lys Lys
   385                 390                 395                 400
   Val Glu Ser Glu Lys Asp Thr Ile Leu Asn Ser Leu Pro Gly Phe Ser
                   405                 410                 415
   Asp Ser Asp Lys Asp Thr Ile Phe Lys Thr Gln Asn Gly Met Met Val
   420 425 430
   Arg Asn Asn Asn Leu Ser Phe Gln Pro Ser Ser Asn Asn Leu Gln Leu
   435 440 445
   Val Gln Asn Gln Asn Ser Gln Ala Ser Asn Gly Ile Ala Asp Pro Asn
   450 455 460
   Phe Ser Asn Val Gln Thr Ala Tyr Asn Lys Ile His Gln Ser Asn Asn
   465 470 475 480
   Thr Pro Glu Lys Thr Leu Asp Ala Val Tyr Ala Ala Val Leu Asp Gln
   485 490 495
   Trp Arg Ser Ile Phe Gln Glu Asp Leu Val Lys Lys Thr Val Asp Leu
   500 505 510
   Leu Glu Lys Tyr Arg Asp His Phe Leu Lys Ala Thr Ala Phe Asn Asn
   515 520 525
   Ile Asp Tyr Ser Lys Gln Asn Ile Ala Ile Ala Asn Asn Val Ser Ser
   530 535 540
   Ala Glu Ser Ala Ser Phe Leu Val Ser Asn Lys Asp Glu Gln Arg Tyr
   545 550 555 560
   Asn Asp Leu Ser Leu Ile Asp Gly Val Asp Leu Lys Ser Trp Leu Phe
   565 570 575
   Lys Pro Glu Gln Asn Glu Ser Asn Pro Leu Asp Thr Ile Tyr Gly Gly
   580 585 590
   Gln Asp Ala Asn Asn Gly Phe Leu Gln Lys Ile Asp Tyr Glu Phe Lys
   595 600 605
   Pro Ser Thr Ser Ser Gly Gly Met Thr Ala Ser Leu Lys Asn Thr Gln
   610 615 620
   Ala Leu Ser Pro Lys Ser Thr Lys Phe Pro Ile Tyr Pro Lys Leu Ala
   625 630 635 640
   Asn Ile Ile Ala Gln Ala Gln Leu Pro Glu Ala Thr Asn Ile Pro Thr
   645 650 655
   Thr Ala Leu Asp Ala Leu Lys Gln Trp Thr Asn Leu Asp Ala Asn Gly
   660 665 670
   Phe Asn Asn Leu Lys Glu Glu Asp Lys Arg Lys Ala Ala Asn Asn Tyr
           675                 680                 685
   Leu Ala Leu Leu Ser Tyr Phe Thr Pro Ala Phe Gln Asp Pro Asn Glu
       690                 695                 700
   Leu Ile Glu Thr Asn Arg Gln Met Leu Glu Ile Pro Ile Thr Val Lys
   705                 710                 715                 720
   Asn Gly Val Asn Pro Leu Ile Leu Pro Thr Asp Gln Gln Asn Leu Val
                   725                 730                 735
   Val Gln Thr Pro Glu Ala His Gly Ala Val Val Ser Gln Gln Trp Leu
               740                 745                 750
   Phe Arg His Asn Lys Glu Ile Leu Pro Gln Glu Gly Glu Tyr Ala Trp
           755                 760                 765
   Lys Thr Ala Leu Gln Thr Pro Asn Asn Phe Pro Asn Trp Leu Asn Asp
       770                 775                 780
   Leu Pro Asp Arg Tyr Lys Phe Ser Ile Asn Gly Leu Thr Phe Ala Ile
   785                 790                 795                 800
   Leu Gly Ile Gly Glu Ser Val Glu Thr Gly Tyr Pro Val Leu Ser Leu
                   805                 810                 815
   Gln Ser Pro Leu Pro Asn Thr Gln Asp Glu Ala Leu Ile Phe Val Asn
               820                 825                 830
   Asp Gln Ala Tyr Arg Ser Ile Leu Phe Ala Val Pro Ala Ala Asn Gln
           835                 840                 845
   Glu Asn Tyr Tyr Ala Phe Lys Ser Thr Asp Leu Lys Gln His Thr Asp
       850                 855                 860
   Gln Asp Pro Val Gln Phe Ile Ala Asn Arg Leu Glu Gly Tyr Leu Asp
   865                 870                 875                 880
   Val Pro Arg Ser Asp Leu Ala Phe Asn Val Lys Asp Ile Ser Lys Phe
                   885                 890                 895
   Asn Tyr Leu Thr Thr Ala Arg Asn Tyr Phe Pro Asp Leu Val Gln Ser
               900                 905                 910
   Tyr Leu Ala Ile Val Ser Thr Val Ile Ala Ile Phe Leu Ile Ile Leu
           915                 920                 925
   Ala Leu Tyr Leu Ile Ile Leu Leu Ile Lys Ser Phe Ile Lys Lys Asn
       930                 935                 940
   Gln Thr Glu Phe Ser Ile Ile Arg Ala Gly Gly Phe Ser Thr Thr Lys
   945                 950                 955                 960
   Phe Ile Val Gly Met Ser Val Phe Ala Gly Ile Val Ala Ile Val Ser
                   965                 970                 975
   Ser Phe Leu Gly Val Leu Phe Ala Phe Leu Leu Glu Gly Gln Val Lys
               980                 985                 990
   Gly Ile Ile Asn Arg Tyr Trp Phe  Ile Ala Leu Pro Glu  Asn Ser Phe
           995                 1000                 1005
   Asn Trp  Leu Ser Phe Phe Gly  Ser Phe Phe Ile Thr  Phe Phe Val
       1010                 1015                 1020
   Phe Glu  Phe Ile Ser Trp Ile  Ala Phe Lys Gln Leu  Phe Ser Lys
       1025                 1030                 1035
   Pro Val  Asn Val Leu Ile Asp  Gln Gly Asn Glu Thr  Lys Phe Ser
       1040                 1045                 1050
   Val Leu  Leu His Leu Leu Lys  His Lys Ser His Thr  Met Ser Pro
       1055                 1060                 1065
   Leu Thr  Lys Phe Arg Val Ser  Leu Ile Val Ser Arg  Phe Ser Arg
       1070                 1075                 1080
   Leu Phe  Thr Tyr Val Gly Leu  Ser Ser Val Ala Leu  Leu Leu Ile
       1085                 1090                 1095
   Gly Ile  Ala Gly Thr Ile Pro  Gln Lys Phe Ser Ala  Ala Gln Thr
       1100                 1105                 1110
   Ser Thr  Ser Leu Asn Arg Asn  Phe Asn Tyr Lys Leu  Asn Leu Gln
       1115                 1120                 1125
   Thr Pro  Thr Glu Gln Ser Gly  Trp Tyr Ala Ile Gln  Pro Tyr Ser
       1130                 1135                 1140
   His Phe  Gly Val Thr Asp Asn  Asn Asn Gly Ile Lys  Thr Leu Tyr
       1145                 1150                 1155
   Asn Glu  Ser Val Gln Ala Asn  Ser Gln Asn Glu His  Pro Tyr Lys
       1160                 1165                 1170
   Pro Ser Asn Leu Lys Leu Lys Asn Arg Gln Asp Gln Pro Ile Lys
   1175 1180 1185
   Ala Ala Asp Gly Thr Glu Leu Glu Leu Gly Asn Leu Leu Leu Pro
   1190 1195 1200
   Ser Tyr Gly Gly Ala Gln Gln Leu Asn Thr Asp Glu Asn Phe Phe
   1205 1210 1215
   Arg His Ala Ser Leu Ser Lys Trp Ile Ile Asp Phe Pro Ile Arg
   1220 1225 1230
   Val Gly Gly Ser Asn Ile Asn Pro Trp Glu Ile Val Glu Lys Ser
   1235 1240 1245
   Ile Pro Lys Gln Ile Thr Gln Leu Leu Ser Ala Ser Ser Asp Gln
   1250 1255 1260
   Phe Leu Ile Ser Val Leu Thr Asp Asp Phe Phe Asn Asn Leu Asn
   1265 1270 1275
   Ala Asn Gly Phe Leu Ile Arg Asn Pro Arg Thr Asn Gln Ile Gln
   1280 1285 1290
   Leu Asp Ala Ser Arg Val Leu Thr Thr Ile Asp Val Phe Asn Pro
   1295 1300 1305
   Gly Gly Val Lys Phe Asn Asp Ser Phe Leu Ser Phe Met Leu Lys
   1310 1315 1320
   Val Tyr Gly Asp Phe Glu Leu Ala Lys Gln Asp Ser Lys Leu Asn
   1325 1330 1335
   Phe Gly Ile Val Pro Val Asp Pro Ala Ile Glu Glu Thr Tyr Thr
   1340 1345 1350
   Tyr Val Glu Gly Pro Phe Gly Phe Gln Glu Asp Asn Leu Asp Glu
   1355 1360 1365
   Asn Ser Pro Tyr Thr Leu Thr Gly Ile Asn Pro Glu Ser Ser Phe
   1370 1375 1380
   Val Asn Leu Ile Asp Gly Ser Gly Asn Ser Leu Arg Asn Leu Ile
   1385 1390 1395
   Ser Ser Asp Gln Glu Met Asn Val Ile Val Asn Ala Gly Phe Gln
   1400 1405 1410
   Tyr Ala Asn Asn Ile Asn Ile Gly Asp Tyr Val Tyr Ile Lys Pro
   1415 1420 1425
   Lys Asn Thr Ala Thr Arg Tyr Ser Glu Lys Phe Leu Lys Ala Pro
   1430 1435 1440
   Leu Asn Asn Ser Thr Val Ala Phe Lys Val Val Gly Val Ser Thr
   1445 1450 1455
   Asp Ala Phe Gly Gln Glu Leu Tyr Ile Asn Gln His Ile Ala Asn
   1460 1465 1470
   Asn Leu Leu Lys Leu Ser Gly Asn Gln Gly Arg Gly Ile Ile Arg
   1475 1480 1485
   Asp Val Ile Lys Lys Thr Asn Gly Gln Ser Gln Ser Ser Asp Glu
   1490 1495 1500
   Tyr Glu Ile Asp Tyr Val Lys Pro Asn Gly Tyr Val Pro Phe Asn
   1505 1510 1515
   Gly Val Phe Ser Lys Glu Leu Lys Pro Ser Leu Leu Asn Lys Ala
   1520 1525 1530
   Leu Val Leu Asn Ser Ile Ile Gly Val Trp Gly Asn Phe Thr Asp
   1535 1540 1545
   Phe Gly Asn Asn Phe Gln Asn Leu Val Arg Asn Lys Leu Asp Lys
   1550 1555 1560
   Val Ile Thr Ser Ile Leu Pro Thr Asp Pro Glu Ile Ile Asn Lys
   1565 1570 1575
   Leu Ala Gln Glu Lys Gln Ile Ile Asn Thr Thr Ser Met Asn Tyr
   1580 1585 1590
   Glu Ser Leu Arg Lys Glu Leu Val Asn Lys Tyr Lys Thr Glu Trp
   1595 1600 1605
   Asn Ser Val Asn Leu Leu Ser Gln Asn Ala Ser Ser Ile Phe Gly
   1610 1615 1620
   Asn Asn Ile Ile Ala Pro Val Leu Asn Ile Asp Ala Ala Gly Thr
   1625 1630 1635
   Ser Ala Gln Ile Ile Arg Asn Asn Ala Glu Val Leu Phe Asn Thr
       1640                1645                1650
   Val Asn Gln Val Asp Ala Phe Leu Leu Gly Thr Ile Ile Pro Phe
       1655                1660                1665
   Ile Phe Ile Thr Cys Val Val Leu Gly Ile Ser Met Leu Glu Glu
       1670                1675                1680
   Met Lys Arg Ile Phe Ile Ser Leu Lys Ala Ile Gly Tyr Arg Asp
       1685                1690                1695
   Val Gln Asn Leu Ile Ser Leu Leu Thr Phe Phe Ile Pro Ala Phe
       1700                1705                1710
   Val Leu Ala Leu Leu Ile Ser Ile Gly Val Leu Ala Gly Val Leu
       1715                1720                1725
   Ile Gly Ile Gln Ala Val Val Phe Asn Val Ala Gln Val Phe Leu
       1730                1735                1740
   Thr Asn Val Phe Glu Phe Leu Pro Tyr Met Val Gly Ile Val Leu
       1745                1750                1755
   Phe Gly Val Thr Ile Phe Val Ile Gly Ser Tyr Phe Trp Ile Lys
       1760                1765                1770
   Leu Arg Ser Ala Glu Leu Lys Glu Gly Phe
       1775                1780
<210> 2
   <211> 134
   <212> PRT
   <213> artificial sequence
<220>
   <223> synthetic polypeptide
<400> 2
   Met Thr Leu Thr Val Gln Ala Arg Gln Leu Leu Ser Gly Ile Val Gln
   1               5                   10                  15
   Gln Gln Asn Asn Leu Leu Arg Ala Ile Glu Ala Gln Gln His Leu Leu
               20                  25                  30
   Gln Leu Thr Val Trp Gly Ile Lys Gln Leu Gln Ala Arg Ile Leu Ala
           35                  40                  45
   Val Glu Arg Tyr Leu Lys Asp Gln Gln Leu Leu Gly Ile Trp Gly Cys
       50                  55                  60
   Ser Gly Lys Leu Ile Cys Thr Thr Ala Val Pro Trp Asn Ala Ser Trp
   65                  70                  75                  80
   Ser Asn Lys Ser Leu Glu Gln Ile Trp Asn Asn Met Thr Trp Met Glu
                   85                  90                  95
   Trp Asp Arg Glu Ile Asn Asn Tyr Thr Ser Leu Ile His Ser Leu Ile
               100                 105                 110
   Glu Glu Ser Gln Asn Gln Gln Glu Lys Asn Glu Gln Glu Leu Leu Glu
           115                 120                 125
   Leu Asp Lys Trp Ala Ser
       130
<210> 3
   <211> 36
   <212> PRT
   <213> artificial sequence
<220>
   <223> synthetic polypeptide
<400> 3
   Leu Val Gly Leu Arg Ile Val Phe Ala Val Leu Ser Ile Val Asn Arg
   1               5                   10                  15
   Val Arg Gln Gly Tyr Ser Pro Leu Ser Phe Gln Thr His Leu Pro Thr
               20                  25                  30
   Pro Arg Gly Pro
           35
<210> 4
   <211> 445
   <212> PRT
   <213> artificial sequence
<220>
   <223> synthetic polypeptide
<400> 4
   Met Val Arg Asn Asn Asn Leu Ser Phe Gln Pro Ser Ser Asn Asn Leu
   1               5                   10                  15
   Gln Leu Val Gln Asn Gln Asn Ser Gln Ala Ser Asn Gly Ile Ala Asp
               20                  25                  30
   Pro Asn Phe Ser Asn Val Gln Thr Ala Tyr Asn Lys Ile His Gln Ser
           35                  40                  45
   Asn Asn Thr Pro Glu Lys Thr Leu Asp Ala Val Tyr Ala Ala Val Leu
       50                  55                  60
   Asp Gln Trp Arg Ser Ile Phe Gln Glu Asp Leu Val Lys Lys Thr Val
   65                  70                  75                  80
   Asp Leu Leu Glu Lys Tyr Arg Asp His Phe Leu Lys Ala Thr Ala Phe
                   85                  90                  95
   Asn Asn Ile Asp Tyr Ser Lys Gln Asn Ile Ala Ile Ala Asn Asn Val
               100                 105                 110
   Ser Ser Ala Glu Ser Ala Ser Phe Leu Val Ser Asn Lys Asp Glu Gln
           115                 120                 125
   Arg Tyr Asn Asp Leu Ser Leu Ile Asp Gly Val Asp Leu Lys Ser Trp
       130                 135                 140
   Leu Phe Lys Pro Glu Gln Asn Glu Ser Asn Pro Leu Asp Thr Ile Tyr
   145                 150                 155                 160
   Gly Gly Gln Asp Ala Asn Asn Gly Phe Leu Gln Lys Ile Asp Tyr Glu
                   165                 170                 175
   Phe Lys Pro Ser Thr Ser Ser Gly Gly Met Thr Ala Ser Leu Lys Asn
               180                 185                 190
   Thr Gln Ala Leu Ser Pro Lys Ser Thr Lys Phe Pro Ile Tyr Pro Lys
           195                 200                 205
   Leu Ala Asn Ile Ile Ala Gln Ala Gln Leu Pro Glu Ala Thr Asn Ile
       210                 215                 220
   Pro Thr Thr Ala Leu Asp Ala Leu Lys Gln Trp Thr Asn Leu Asp Ala
   225                 230                 235                 240
   Asn Gly Phe Asn Asn Leu Lys Glu Glu Asp Lys Arg Lys Ala Ala Asn
                   245                 250                 255
   Asn Tyr Leu Ala Leu Leu Ser Tyr Phe Thr Pro Ala Phe Gln Asp Pro
               260                 265                 270
   Asn Glu Leu Ile Glu Thr Asn Arg Gln Met Leu Glu Ile Pro Ile Thr
           275                 280                 285
   Val Lys Asn Gly Val Asn Pro Leu Ile Leu Pro Thr Asp Gln Gln Asn
       290                 295                 300
   Leu Val Val Gln Thr Pro Glu Ala His Gly Ala Val Val Ser Gln Gln
   305                 310                 315                 320
   Trp Leu Phe Arg His Asn Lys Glu Ile Leu Pro Gln Glu Gly Glu Tyr
                   325                 330                 335
   Ala Trp Lys Thr Ala Leu Gln Thr Pro Asn Asn Phe Pro Asn Trp Leu
               340                 345                 350
   Asn Asp Leu Pro Asp Arg Tyr Lys Phe Ser Ile Asn Gly Leu Thr Phe
           355                 360                 365
   Ala Ile Leu Gly Ile Gly Glu Ser Val Glu Thr Gly Tyr Pro Val Leu
       370                 375                 380
   Ser Leu Gln Ser Pro Leu Pro Asn Thr Gln Asp Glu Ala Leu Ile Phe
   385                 390                 395                 400
   Val Asn Asp Gln Ala Tyr Arg Ser Ile Leu Phe Ala Val Pro Ala Ala
                   405                 410                 415
   Asn Gln Glu Asn Tyr Tyr Ala Phe Lys Ser Thr Asp Leu Lys Gln His
               420                 425                 430
   Thr Asp Gln Asp Pro Val Gln Phe Ile Ala Asn Arg Leu
           435                 440                 445
<210> 5
   <211> 1338
   <212> DNA
   <213> artificial sequence
<220>
   <223> synthetic polynucleotide
<400> 5
   atggtgcgca ataataatct gagctttcag ccgagcagca ataatctgca gctggtgcag 60
   aatcagaata gccaggcaag caatggtatt gcagatccga attttagcaa tgttcagacc 120
   gcctataata aaattcatca gagcaataat acaccggaaa aaaccctgga tgcagtttat 180
   gcagcagttc tggatcagtg gcgtagcatt tttcaggaag atctggttaa aaaaaccgtg 240
   gatctgctgg aaaaatatcg cgatcatttt ctgaaagcca ccgcctttaa taatattgat 300
   tatagcaaac agaatattgc cattgccaat aatgttagca gcgcagaaag cgcaagcttt 360
   ctggtgagca ataaagatga acagcgctat aatgatctga gcctgattga tggtgttgat 420
   ctgaaaagct ggctgtttaa accggaacag aatgaaagca atccgctgga taccatttat 480
   ggtggtcagg atgccaataa tggctttctg cagaaaattg attatgaatt taaaccgagc 540
   accagcagcg gtggtatgac cgcaagcctg aaaaataccc aggcactgag cccgaaaagc 600
   accaaatttc cgatttatcc gaaactggcc aatattattg cacaggcaca gctgccggaa 660
   gcaaccaata ttccgaccac cgcactggat gcactgaaac agtggaccaa tctggatgcc 720
   aatggcttta ataatctgaa agaagaagat aaacgcaaag cagccaataa ttatctggca 780
   ctgctgagct attttacacc ggcatttcag gatccgaatg aactgattga aaccaatcgt 840
   cagatgctgg aaattccgat taccgtgaaa aatggtgtga atccgctgat cctgccgacc 900
   gatcagcaga atctggttgt tcagacaccg gaagcacatg gtgcagttgt tagccagcag 960
   tggctgtttc gtcataataa agaaattctg ccgcaggaag gtgaatatgc atggaaaacc 1020
   gcactgcaga ccccgaataa ttttccgaat tggctgaatg atctgccgga tcgctataaa 1080
   tttagcatta atggcctgac ctttgcaatt ctgggtattg gtgaaagcgt tgaaaccggt 1140
   tatccggttc tgagcctgca gtctccgctg ccgaataccc aggatgaagc cctgattttt 1200
   gttaatgatc aggcctatcg cagcattctg tttgcagttc cggcagcaaa tcaggaaaat 1260
   tattacgcct ttaaaagcac cgatctgaaa cagcataccg atcaggatcc ggttcagttt 1320
   attgcaaatc gcctgtaa 1338
<210> 6
   <211> 1359
   <212> DNA
   <213> artificial sequence
<220>
   <223> synthetic polynucleotide
<400> 6
   atggtgcgca ataataatct gagctttcag ccgagcagca ataatctgca gctggtgcag      60
   aatcagaata gccaggcaag caatggtatt gcagatccga attttagcaa tgttcagacc     120
   gcctataata aaattcatca gagcaataat acaccggaaa aaaccctgga tgcagtttat     180
   gcagcagttc tggatcagtg gcgtagcatt tttcaggaag atctggttaa aaaaaccgtg     240
   gatctgctgg aaaaatatcg cgatcatttt ctgaaagcca ccgcctttaa taatattgat     300
   tatagcaaac agaatattgc cattgccaat aatgttagca gcgcagaaag cgcaagcttt     360
   ctggtgagca ataaagatga acagcgctat aatgatctga gcctgattga tggtgttgat     420
   ctgaaaagct ggctgtttaa accggaacag aatgaaagca atccgctgga taccatttat     480
   ggtggtcagg atgccaataa tggctttctg cagaaaattg attatgaatt taaaccgagc     540
   accagcagcg gtggtatgac cgcaagcctg aaaaataccc aggcactgag cccgaaaagc     600
   accaaatttc cgatttatcc gaaactggcc aatattattg cacaggcaca gctgccggaa     660
   gcaaccaata ttccgaccac cgcactggat gcactgaaac agtggaccaa tctggatgcc     720
   aatggcttta ataatctgaa agaagaagat aaacgcaaag cagccaataa ttatctggca     780
   ctgctgagct attttacacc ggcatttcag gatccgaatg aactgattga aaccaatcgt     840
   cagatgctgg aaattccgat taccgtgaaa aatggtgtga atccgctgat cctgccgacc     900
   gatcagcaga atctggttgt tcagacaccg gaagcacatg gtgcagttgt tagccagcag     960
   tggctgtttc gtcataataa agaaattctg ccgcaggaag gtgaatatgc atggaaaacc    1020
   gcactgcaga ccccgaataa ttttccgaat tggctgaatg atctgccgga tcgctataaa    1080
   tttagcatta atggcctgac ctttgcaatt ctgggtattg gtgaaagcgt tgaaaccggt    1140
   tatccggttc tgagcctgca gtctccgctg ccgaataccc aggatgaagc cctgattttt    1200
   gttaatgatc aggcctatcg cagcattctg tttgcagttc cggcagcaaa tcaggaaaat    1260
   tattacgcct ttaaaagcac cgatctgaaa cagcataccg atcaggatcc ggttcagttt    1320
   attgcaaatc gcctgcatca ccatcaccat cattaataa                           1359
<210> 7
   <211> 451
   <212> PRT
   <213> artificial sequence
<220>
   <223> synthetic polypeptide
<400> 7
   Met Val Arg Asn Asn Asn Leu Ser Phe Gln Pro Ser Ser Asn Asn Leu
   1               5                   10                  15
   Gln Leu Val Gln Asn Gln Asn Ser Gln Ala Ser Asn Gly Ile Ala Asp
               20                  25                  30
   Pro Asn Phe Ser Asn Val Gln Thr Ala Tyr Asn Lys Ile His Gln Ser
           35                  40                  45
   Asn Asn Thr Pro Glu Lys Thr Leu Asp Ala Val Tyr Ala Ala Val Leu
       50                  55                  60
   Asp Gln Trp Arg Ser Ile Phe Gln Glu Asp Leu Val Lys Lys Thr Val
   65                  70                  75                  80
   Asp Leu Leu Glu Lys Tyr Arg Asp His Phe Leu Lys Ala Thr Ala Phe
                   85                  90                  95
   Asn Asn Ile Asp Tyr Ser Lys Gln Asn Ile Ala Ile Ala Asn Asn Val
               100                 105                 110
   Ser Ser Ala Glu Ser Ala Ser Phe Leu Val Ser Asn Lys Asp Glu Gln
           115                 120                 125
   Arg Tyr Asn Asp Leu Ser Leu Ile Asp Gly Val Asp Leu Lys Ser Trp
       130                 135                 140
   Leu Phe Lys Pro Glu Gln Asn Glu Ser Asn Pro Leu Asp Thr Ile Tyr
   145                 150                 155                 160
   Gly Gly Gln Asp Ala Asn Asn Gly Phe Leu Gln Lys Ile Asp Tyr Glu
                   165                 170                 175
   Phe Lys Pro Ser Thr Ser Ser Gly Gly Met Thr Ala Ser Leu Lys Asn
               180                 185                 190
   Thr Gln Ala Leu Ser Pro Lys Ser Thr Lys Phe Pro Ile Tyr Pro Lys
           195                 200                 205
   Leu Ala Asn Ile Ile Ala Gln Ala Gln Leu Pro Glu Ala Thr Asn Ile
       210                 215                 220
   Pro Thr Thr Ala Leu Asp Ala Leu Lys Gln Trp Thr Asn Leu Asp Ala
   225                 230                 235                 240
   Asn Gly Phe Asn Asn Leu Lys Glu Glu Asp Lys Arg Lys Ala Ala Asn
                   245                 250                 255
   Asn Tyr Leu Ala Leu Leu Ser Tyr Phe Thr Pro Ala Phe Gln Asp Pro
               260                 265                 270
   Asn Glu Leu Ile Glu Thr Asn Arg Gln Met Leu Glu Ile Pro Ile Thr
           275                 280                 285
   Val Lys Asn Gly Val Asn Pro Leu Ile Leu Pro Thr Asp Gln Gln Asn
       290                 295                 300
   Leu Val Val Gln Thr Pro Glu Ala His Gly Ala Val Val Ser Gln Gln
   305                 310                 315                 320
   Trp Leu Phe Arg His Asn Lys Glu Ile Leu Pro Gln Glu Gly Glu Tyr
                   325                 330                 335
   Ala Trp Lys Thr Ala Leu Gln Thr Pro Asn Asn Phe Pro Asn Trp Leu
               340                 345                 350
   Asn Asp Leu Pro Asp Arg Tyr Lys Phe Ser Ile Asn Gly Leu Thr Phe
   355 360 365
   Ala Ile Leu Gly Ile Gly Glu Ser Val Glu Thr Gly Tyr Pro Val Leu
   370 375 380
   Ser Leu Gln Ser Pro Leu Pro Asn Thr Gln Asp Glu Ala Leu Ile Phe
   385 390 395 400
   Val Asn Asp Gln Ala Tyr Arg Ser Ile Leu Phe Ala Val Pro Ala Ala
   405 410 415
   Asn Gln Glu Asn Tyr Tyr Ala Phe Lys Ser Thr Asp Leu Lys Gln His
   420 425 430
   Thr Asp Gln Asp Pro Val Gln Phe Ile Ala Asn Arg Leu His His His
   435 440 445
   His His His
   450
<210> 8
   <211> 110
   <212> PRT
   <213> artificial sequence
<220>
   <223> synthetic polypeptide
<400> 8
   Glu Val Gln Met Val Glu Ser Gly Gly Gly Leu Val Lys Pro Gly Gly
   1               5                   10                  15
   Ser Leu Lys Leu Ser Cys Ala Ala Ser Gly Phe Ala Phe Asn Lys Tyr
               20                  25                  30
   Asp Met Ser Trp Val Arg Gln Thr Pro Ala Lys Arg Leu Glu Trp Val
           35                  40                  45
   Ala Tyr Ile Ser Gly Gly Gly Gly His Thr Tyr Tyr Arg Asp Thr Leu
       50                  55                  60
   Lys Gly Arg Phe Thr Val Ser Arg Asp Asn Ala Lys Asn Thr Leu Tyr
   65                  70                  75                  80
   Leu Gln Met Asn Ser Leu Lys Ser Glu Asp Thr Ala Met Tyr Tyr Cys
                   85                  90                  95
   Thr Arg His Gly Thr Ser Trp Asp Tyr Trp Gly Gln Gly Thr
               100                 105                 110
<210> 9
   <211> 107
   <212> PRT
   <213> artificial sequence
<220>
   <223> synthetic polypeptide
<400> 9
   Asp Ile Gln Met Asn Gln Ser Pro Ser Ser Leu Ser Ala Ser Leu Gly
   1               5                   10                  15
   Asp Thr Ile Ser Ile Thr Cys Arg Ala Ser Gln Asn Ile Asn Phe Trp
               20                  25                  30
   Leu Ser Trp Tyr Gln Leu Lys Pro Gly Asn Ile Pro Lys Gln Leu Ile
           35                  40                  45
   Tyr Lys Thr Ser Asn Leu His Thr Gly Val Pro Ser Arg Phe Ser Gly
       50                  55                  60
   Ser Gly Ser Gly Thr Asp Phe Thr Leu Thr Ile Ser Ser Leu Gln Pro
   65                  70                  75                  80
   Glu Asp Ile Ala Thr Tyr Tyr Cys Leu Gln Gly Gln Ser Tyr Pro Trp
                   85                  90                  95
   Thr Phe Gly Gly Gly Thr Lys Leu Glu Ile Lys
               100                 105
<210> 10
   <211> 353
   <212> PRT
   <213> artificial sequence
<220>
   <223> synthetic polypeptide
<400> 10
   Ala Ser Pro Thr Ser Pro Lys Val Phe Pro Leu Ser Leu Cys Ser Thr
   1               5                   10                  15
   Gln Pro Asp Gly Asn Val Val Ile Ala Cys Leu Val Gln Gly Phe Phe
               20                  25                  30
   Pro Gln Glu Pro Leu Ser Val Thr Trp Asn Lys Ser Gly Gln Gly Val
           35                  40                  45
   Thr Ala Arg Asn Phe Pro Pro Ser Gln Asp Ala Ser Gly Asp Leu Tyr
           50                  55                  60
   Thr Thr Ser Ser Gln Leu Thr Leu Pro Ala Thr Gln Cys Leu Ala Gly
   65                  70                  75                  80
   Lys Ser Val Thr Cys His Val Lys His Tyr Thr Asn Pro Ser Gln Asp
                   85                  90                  95
   Val Thr Val Pro Cys Pro Val Pro Ser Thr Pro Pro Thr Pro Ser Pro
               100                 105                 110
   Ser Thr Pro Pro Thr Pro Ser Pro Ser Cys Cys His Pro Arg Leu Ser
           115                 120                 125
   Leu His Arg Pro Ala Leu Glu Asp Leu Leu Leu Gly Ser Glu Ala Asn
       130                 135                 140
   Leu Thr Cys Thr Leu Thr Gly Leu Arg Asp Ala Ser Gly Val Thr Phe
   145                 150                 155                 160
   Thr Trp Thr Pro Ser Ser Gly Lys Ser Ala Val Gln Gly Pro Pro Asp
                   165                 170                 175
   Arg Asp Leu Cys Gly Cys Tyr Ser Val Ser Ser Val Leu Ser Gly Cys
               180                 185                 190
   Ala Glu Pro Trp Asn His Gly Lys Thr Phe Thr Cys Thr Ala Ala Tyr
           195                 200                 205
   Pro Glu Ser Lys Thr Pro Leu Thr Ala Thr Leu Ser Lys Ser Gly Asn
       210                 215                 220
   Thr Phe Arg Pro Glu Val His Leu Leu Pro Pro Pro Ser Glu Glu Leu
   225                 230                 235                 240
   Ala Leu Asn Glu Leu Val Thr Leu Thr Cys Leu Ala Arg Gly Phe Ser
                   245                 250                 255
   Pro Lys Asp Val Leu Val Arg Trp Leu Gln Gly Ser Gln Glu Leu Pro
               260                 265                 270
   Arg Glu Lys Tyr Leu Thr Trp Ala Ser Arg Gln Glu Pro Ser Gln Gly
           275                 280                 285
   Thr Thr Thr Phe Ala Val Thr Ser Ile Leu Arg Val Ala Ala Glu Asp
       290                 295                 300
   Trp Lys Lys Gly Asp Thr Phe Ser Cys Met Val Gly His Glu Ala Leu
   305 310 315 320
   Pro Leu Ala Phe Thr Gln Lys Thr Ile Asp Arg Leu Ala Gly Lys Pro
   325 330 335
   Thr His Val Asn Val Ser Val Val Met Ala Glu Val Asp Gly Thr Cys
   340 345 350
   Tyr
<210> 11
   <211> 106
   <212> PRT
   <213> artificial sequence
<220>
   <223> synthetic polypeptide
<400> 11
   Ala Asp Ala Ala Pro Thr Val Ser Ile Phe Pro Pro Ser Ser Glu Gln
   1 5 10 15
   Leu Thr Ser Gly Gly Ala Ser Val Val Cys Phe Leu Asn Asn Phe Tyr
   20 25 30
   Pro Lys Asp Ile Asn Val Lys Trp Lys Ile Asp Gly Ser Glu Arg Gln
   35 40 45
   Asn Gly Val Leu Asn Ser Trp Thr Asp Gln Asp Ser Lys Asp Ser Thr
   50 55 60
   Tyr Ser Met Ser Ser Thr Leu Thr Leu Thr Lys Asp Glu Tyr Glu Arg
   65 70 75 80
   His Asn Ser Tyr Thr Cys Glu Ala Thr His Lys Thr Ser Thr Ser Pro
   85 90 95
   Ile Val Lys Ser Phe Asn Arg Asn Glu Cys
   100 105
<210> 12
   <211> 415
   <212> DNA
   <213> artificial sequence
<220>
   <223> synthetic polynucleotide
<400> 12
   actagaattc atgaactttg ggctcagctt ggttttcctt gtccttactt taaaaggtgt        60
   gaagtgtgaa gtccagatgg tggagtctgg gggaggctta gtgaagcctg gagggtccct       120
   gaaactctcc tgtgcagcct ctggattcgc tttcaacaaa tatgacatgt cttgggttcg       180
   ccagactccg gcgaagaggc tggagtgggt cgcatacatt agtggtggtg gtggtcatac       240
   ttactatcga gacactttga agggccgctt caccgtctcc agagacaatg ccaagaacac       300
   cctataccta caaatgaaca gtctgaagtc tgaagacaca gccatgtatt actgtacaag       360
   acacggtact agctgggact actggggcca aggcaccgct ctcactgtct cctca            415
<210> 13
   <211> 394
   <212> DNA
   <213> artificial sequence
<220>
   <223> synthetic polynucleotide
<400> 13
   actatgtaca atgagtgtgc tcactcaggt cctggcgttg ctgctgttct gctttttagg        60
   tgtgagatgt gacatccaga tgaaccagtc tccatccagt ctgtctgcat ccctcggaga       120
   cacaatttcc atcacttgcc gtgccagtca gaacattaat ttttggttga gctggtacca       180
   gctgaaacca ggaaatattc ctaaacaatt gatctataag acttccaact tgcacacagg       240
   cgtcccatca aggtttagtg gcagtggatc tggaacagat ttcacattaa ccatcagcag       300
   tctgcagcct gaagacattg ccacttacta ctgtctccag ggtcagagtt atccgtggac       360
   gttcggtggg ggcaccaaac tggaaatcaa acgg                                   394
<210> 14
   <211> 861
   <212> PRT
   <213> Human immunodeficiency virus
<400> 14
   Met Arg Val Lys Glu Lys Tyr Gln His Leu Trp Arg Trp Gly Trp Lys
   1               5                   10                  15
   Trp Gly Thr Met Leu Leu Gly Ile Leu Met Ile Cys Ser Ala Thr Glu
               20                  25                  30
   Lys Leu Trp Val Thr Val Tyr Tyr Gly Val Pro Val Trp Lys Glu Ala
           35                  40                  45
   Thr Thr Thr Leu Phe Cys Ala Ser Asp Ala Lys Ala Tyr Asp Thr Glu
       50                  55                  60
   Val His Asn Val Trp Ala Thr His Ala Cys Val Pro Thr Asp Pro Asn
   65 70 75 80
   Pro Gln Glu Val Val Leu Val Asn Val Thr Glu Asn Phe Asn Met Trp
   85 90 95
   Lys Asn Asp Met Val Glu Gln Met His Glu Asp Ile Ile Ser Leu Trp
   100 105 110
   Asp Gln Ser Leu Lys Pro Cys Val Lys Leu Thr Pro Leu Cys Val Ser
   115 120 125
   Leu Lys Cys Thr Asp Leu Gly Asn Ala Thr Asn Thr Asn Ser Ser Asn
   130 135 140
   Thr Asn Ser Ser Ser Gly Glu Met Met Met Glu Lys Gly Glu Ile Lys
   145 150 155 160
   Asn Cys Ser Phe Asn Ile Ser Thr Ser Ile Arg Gly Lys Val Gln Lys
   165 170 175
   Glu Tyr Ala Phe Phe Tyr Lys Leu Asp Ile Ile Pro Ile Asp Asn Asp
   180 185 190
   Thr Thr Ser Tyr Thr Leu Thr Ser Cys Asn Thr Ser Val Ile Thr Gln
   195 200 205
   Ala Cys Pro Lys Val Ser Phe Glu Pro Ile Pro Ile His Tyr Cys Ala
   210 215 220
   Pro Ala Gly Phe Ala Ile Leu Lys Cys Asn Asn Lys Thr Phe Asn Gly
   225 230 235 240
   Thr Gly Pro Cys Thr Asn Val Ser Thr Val Gln Cys Thr His Gly Ile
   245 250 255
   Arg Pro Val Val Ser Thr Gln Leu Leu Leu Asn Gly Ser Leu Ala Glu
   260 265 270
   Glu Glu Val Val Ile Arg Ser Ala Asn Phe Thr Asp Asn Ala Lys Thr
   275 280 285
   Ile Ile Val Gln Leu Asn Gln Ser Val Glu Ile Asn Cys Thr Arg Pro
   290 295 300
   Asn Asn Asn Thr Arg Lys Ser Ile Arg Ile Gln Arg Gly Pro Gly Arg
   305 310 315 320
   Ala Phe Val Thr Ile Gly Lys Ile Gly Asn Met Arg Gln Ala His Cys
                   325                 330                 335
   Asn Ile Ser Arg Ala Lys Trp Asn Ala Thr Leu Lys Gln Ile Ala Ser
               340                 345                 350
   Lys Leu Arg Glu Gln Phe Gly Asn Asn Lys Thr Ile Ile Phe Lys Gln
           355                 360                 365
   Ser Ser Gly Gly Asp Pro Glu Ile Val Thr His Ser Phe Asn Cys Gly
       370                 375                 380
   Gly Glu Phe Phe Tyr Cys Asn Ser Thr Gln Leu Phe Asn Ser Thr Trp
   385                 390                 395                 400
   Phe Asn Ser Thr Trp Ser Thr Glu Gly Ser Asn Asn Thr Glu Gly Ser
                   405                 410                 415
   Asp Thr Ile Thr Leu Pro Cys Arg Ile Lys Gln Phe Ile Asn Met Trp
               420                 425                 430
   Gln Glu Val Gly Lys Ala Met Tyr Ala Pro Pro Ile Ser Gly Gln Ile
           435                 440                 445
   Arg Cys Ser Ser Asn Ile Thr Gly Leu Leu Leu Thr Arg Asp Gly Gly
       450                 455                 460
   Asn Asn Asn Asn Gly Ser Glu Ile Phe Arg Pro Gly Gly Gly Asp Met
   465                 470                 475                 480
   Arg Asp Asn Trp Arg Ser Glu Leu Tyr Lys Tyr Lys Val Val Lys Ile
                   485                 490                 495
   Glu Pro Leu Gly Val Ala Pro Thr Lys Ala Lys Arg Arg Val Val Gln
               500                 505                 510
   Arg Glu Lys Arg Ala Val Gly Ile Gly Ala Leu Phe Leu Gly Phe Leu
           515                 520                 525
   Gly Ala Ala Gly Ser Thr Met Gly Ala Arg Ser Met Thr Leu Thr Val
       530                 535                 540
   Gln Ala Arg Gln Leu Leu Ser Gly Ile Val Gln Gln Gln Asn Asn Leu
   545                 550                 555                 560
   Leu Arg Ala Ile Glu Ala Gln Gln His Leu Leu Gln Leu Thr Val Trp
                   565                 570                 575
   Gly Ile Lys Gln Leu Gln Ala Arg Ile Leu Ala Val Glu Arg Tyr Leu
               580                 585                 590
   Lys Asp Gln Gln Leu Leu Gly Ile Trp Gly Cys Ser Gly Lys Leu Ile
           595                 600                 605
   Cys Thr Thr Ala Val Pro Trp Asn Ala Ser Trp Ser Asn Lys Ser Leu
       610                 615                 620
   Glu Gln Ile Trp Asn Asn Met Thr Trp Met Glu Trp Asp Arg Glu Ile
   625                 630                 635                 640
   Asn Asn Tyr Thr Ser Leu Ile His Ser Leu Ile Glu Glu Ser Gln Asn
                   645                 650                 655
   Gln Gln Glu Lys Asn Glu Gln Glu Leu Leu Glu Leu Asp Lys Trp Ala
               660                 665                 670
   Ser Leu Trp Asn Trp Phe Asn Ile Thr Asn Trp Leu Trp Tyr Ile Lys
           675                 680                 685
   Ile Phe Ile Met Ile Val Gly Gly Leu Val Gly Leu Arg Ile Val Phe
       690                 695                 700
   Ala Val Leu Ser Ile Val Asn Arg Val Arg Gln Gly Tyr Ser Pro Leu
   705                 710                 715                 720
   Ser Phe Gln Thr His Leu Pro Thr Pro Arg Gly Pro Asp Arg Pro Glu
                   725                 730                 735
   Gly Ile Glu Glu Glu Gly Gly Glu Arg Asp Arg Asp Arg Ser Ile Arg
               740                 745                 750
   Leu Val Asn Gly Ser Leu Ala Leu Ile Trp Asp Asp Leu Arg Ser Leu
           755                 760                 765
   Cys Leu Phe Ser Tyr His Arg Leu Arg Asp Leu Leu Leu Ile Val Thr
       770                 775                 780
   Arg Ile Val Glu Leu Leu Gly Arg Arg Gly Trp Glu Ala Leu Lys Tyr
   785                 790                 795                 800
   Trp Trp Asn Leu Leu Gln Tyr Trp Ser Gln Glu Leu Lys Asn Ser Ala
                   805                 810                 815
   Val Ser Leu Leu Asn Ala Thr Ala Ile Ala Val Ala Glu Gly Thr Asp
               820                 825                 830
   Arg Val Ile Glu Val Val Gln Gly Ala Cys Arg Ala Ile Arg His Ile
   835 840 845
   Pro Arg Arg Ile Arg Gln Gly Leu Glu Arg Ile Leu Leu
   850 855 860
<210> 15
   <211> 11
   <212> PRT
   <213> artificial sequence
<220>
   <223> synthetic peptide
<400> 15
   Gly Gly Gly Gly Ser His His His His His His
   1 5 10
<210> 16
   <211> 144
   <212> PRT
   <213> artificial sequence
<220>
   <223> synthetic polynucleotide
<400> 16
   Met Thr Leu Thr Val Gln Ala Arg Gln Leu Leu Ser Gly Ile Val Gln
   1 5 10 15
   Gln Gln Asn Asn Leu Leu Arg Ala Ile Glu Ala Gln Gln His Leu Leu
   20 25 30
   Gln Leu Thr Val Trp Gly Ile Lys Gln Leu Gln Ala Arg Ile Leu Ala
   35 40 45
   Val Glu Arg Tyr Leu Lys Asp Gln Gln Leu Leu Gly Ile Trp Gly Cys
   50 55 60
   Ser Gly Lys Leu Ile Cys Thr Thr Ala Val Pro Trp Asn Ala Ser Trp
   65 70 75 80
   Ser Asn Lys Ser Leu Glu Gln Ile Trp Asn Asn Met Thr Trp Met Glu
   85 90 95
   Trp Asp Arg Glu Ile Asn Asn Tyr Thr Ser Leu Ile His Ser Leu Ile
   100 105 110
   Glu Glu Ser Gln Asn Gln Gln Glu Lys Asn Glu Gln Glu Leu Leu Glu
   115 120 125
   Leu Asp Lys Trp Ala Ser Gly Gly Gly Gly Ser His His His His His
   130 135 140
<210> 17
   <211> 112
   <212> PRT
   <213> artificial sequence
<220>
   <223> synthetic polypeptide
<400> 17
   Gln Val Gln Leu Val Gln Ser Gly Gly Gln Met Lys Lys Pro Gly Glu
   1 5 10 15
   Ser Met Arg Ile Ser Cys Arg Ala Ser Gly Tyr Glu Phe Ile Asp Cys
   20 25 30
   Thr Leu Asn Trp Ile Arg Leu Ala Pro Gly Lys Arg Pro Glu Trp Met
   35 40 45
   Gly Trp Leu Lys Pro Arg Gly Gly Ala Val Asn Tyr Ala Arg Pro Leu
   50 55 60
   Gln Gly Arg Val Thr Met Thr Arg Asp Val Tyr Ser Asp Thr Ala Phe
   65 70 75 80
   Leu Glu Leu Arg Ser Leu Thr Val Asp Asp Thr Ala Val Tyr Phe Cys
   85 90 95
   Thr Arg Gly Lys Asn Cys Asp Tyr Asn Trp Asp Phe Glu His Trp Gly
   100 105 110
<210> 18
   <211> 98
   <212> PRT
   <213> artificial sequence
<220>
   <223> synthetic polypeptide
<400> 18
   Gln Val Gln Leu Val Gln Ser Gly Ala Glu Val Lys Lys Pro Gly Ala
   1 5 10 15
   Ser Val Lys Val Ser Cys Lys Ala Ser Gly Tyr Thr Phe Thr Gly Tyr
   20 25 30
   Tyr Met His Trp Val Arg Gln Ala Pro Gly Gln Gly Leu Glu Trp Met
           35                  40                  45
   Gly Trp Ile Asn Pro Asn Ser Gly Gly Thr Asn Tyr Ala Gln Lys Phe
       50                  55                  60
   Gln Gly Arg Val Thr Met Thr Arg Asp Thr Ser Ile Ser Thr Ala Tyr
   65                  70                  75                  80
   Met Glu Leu Ser Arg Leu Arg Ser Asp Asp Thr Ala Val Tyr Tyr Cys
                   85                  90                  95
   Ala Arg
<210> 19
   <211> 116
   <212> PRT
   <213> artificial sequence
<220>
   <223> synthetic polypeptide
<400> 19
   Glu Val Gln Met Val Glu Ser Gly Gly Gly Leu Val Lys Pro Gly Gly
   1               5                   10                  15
   Ser Leu Lys Leu Ser Cys Ala Ala Ser Gly Phe Ala Phe Asn Lys Tyr
               20                  25                  30
   Asp Met Ser Trp Val Arg Gln Thr Pro Ala Lys Arg Leu Glu Trp Val
           35                  40                  45
   Ala Tyr Ile Ser Gly Gly Gly Gly His Thr Tyr Tyr Arg Asp Thr Leu
       50                  55                  60
   Lys Gly Arg Phe Thr Val Ser Arg Asp Asn Ala Lys Asn Thr Leu Tyr
   65                  70                  75                  80
   Leu Gln Met Asn Ser Leu Lys Ser Glu Asp Thr Ala Met Tyr Tyr Cys
                   85                  90                  95
   Thr Arg His Gly Thr Ser Trp Asp Tyr Trp Gly Gln Gly Thr Ala Leu
               100                 105                 110
   Thr Val Ser Ser
           115
<210> 20
   <211> 121
   <212> PRT
   <213> artificial sequence
<220>
   <223> synthetic polypeptide
<400> 20
   Glu Val Lys Leu Val Glu Ser Gly Gly Gly Leu Val Gln Pro Gly Gly
   1               5                   10                  15
   Ser Leu Lys Leu Ser Cys Ala Ala Ser Gly Phe Thr Phe Ser Asp Tyr
               20                  25                  30
   Tyr Met Tyr Trp Val Arg Gln Thr Pro Glu Lys Arg Leu Glu Trp Val
           35                  40                  45
   Ala Tyr Ile Ser Asn Gly Gly Gly Ser Thr Tyr Tyr Pro Asp Thr Val
       50                  55                  60
   Lys Gly Arg Phe Thr Ile Ser Arg Asp Asn Ala Lys Asn Thr Leu Tyr
   65                  70                  75                  80
   Leu Gln Met Ser Arg Leu Lys Ser Glu Asp Thr Ala Met Tyr Tyr Cys
                   85                  90                  95
   Ala Arg Gly Lys Asn Cys Asp Tyr Asn Trp Asp Phe Glu His Trp Gly
               100                 105                 110
   Arg Gly Thr Pro Val Ile Val Ser Ser
           115                 120

## Description

The invention relates to a new non-HIV vaccine antigen from the vaginal microbiota capable of inducing a mucosal neutralizing protective antibody response against HIV infection and to a neutralizing antibody directed to said antigen.

Despite thirty years of study, there is no HIV vaccine. HIV-exposed uninfected individuals are currently the focus of intense studies because identifying the immune correlates of reduced susceptibility to HIV may offer important clues for the development of a HIV vaccine. It was hypothesized that hypermutated neutralizing monoclonal antibodies, isolated from HIV-infected patients, came from ancestor germinal B-cell lineages, primed initially by self- or non-HIV-1 antigens and boosted for HIV Env specific response by other immunogens (Haynes et al., Nature biotechnology, 2012, 30, 423-33). Along this line, it has been recently suggested to design HIV immunogens that target specific germline B-cell receptors (BCR; Jardine et al., Science, 2013, 340, 711-6). This was achieved by the "reverse-design" of antigens from the native HIV-gp120 CD4-binding site (CD4bs-gp120) epitope to initiate the binding with germline B-cells receptors. As expected, affinity comparison of various hypermutated neutralizing monoclonal antibodies (VRC01, 12A12, 3BNC60, NIH46-45 PGV04, PGV19, PGV20, VRC-CH31) with that of their germline version shows that the affinity of the antibody precursor is 2000 to 20000 times lower for the domain CD4bs-gp120 than that of the hypermutated versions (Hoot, S. *et al.,* PLoS pathogens, 2013, 9, e1003106). Conversely, to determine the original epitope recognized by VRC01, several successive point mutations of CD4bs-gp120 domain were needed to better fit with the germinal BCR (composed by an heavy chain encoded by unmutated hVH1-2^{∗}2). According to the authors, only rare mutation events changing the antigenic nature of gp120 during infection would generate an epitope capable of activating a population of naive B-cells, precursors of a protective antibody response. This population would not be recruited by the native form of the envelope protein. The difficulty of this approach is to trace the historical evolution of viral antigens during the infection in the patient. In addition reverse-designed HIV antigens were not shown to be capable of eliciting HIV neutralizing antibodies by active immunisation.

The transgenic mouse strain HAMIGA (EP patent 1 680 449) is a humanized transgenic mouse strain expressing human/murine chimeric IgAs with human IgA heavy chain constant regions (CH1 to CH3) and mouse heavy chain variable region and light chain variable and constant regions (VH, VL and CL).

Fraser et al. (Science, 1995, 270, 39-403) discloses the complete nucleotide sequence of the *M. genitalium* genome with predicted coding regions including a locus tag MG 468 encoding an hypothetical ABC transporter permease protein (GenBank accession number AAC72488.1). Butt et al. (Infection, Genetics and Evolution, 2012, 112, 53-62) discloses the identification of predicted transmembrane proteins of *M. genitalium* that are of interest for their potential as vaccine candidates against *M. genitalium* infection. The inventors have shown that a monoclonal IgA antibody isolated from humanized HAMIGA transgenic mice immunised with a recombinant truncated permease of *Mycoplasma genitalium* (fragment 431 to 875 of *M. genitalium* ABC transporter permease protein GenBank accession number AAC72488.1 or SEQ ID NO: 1), a potential pathogen of the genital tract, is able to neutralize HIV-1 primary isolates. This is one of the rare HIV-1 neutralizing monoclonal antibody induced by active immunisation (Nishiyama, Y. et al., The Journal of Biological Chemistry, 2009, 284, 30627-30642; Sreepian, A. et al., Journal of Immune based Therapies and Vaccines, 2009, 7, 5) and the first to be elicited by a HIV-1 independent antigen. All the others have been isolated from HIV infected patients (McCoy, L. E. & Weiss, R. A. The Journal of Experimental Medicine, 2013, 210, 209-223). The characteristics of this IgA antibody and the nature of the immunogen suggest that the natural immune response to genital *microbiota* (commensals and/or pathogens) may contribute to the natural resistance to HIV infection.

In view of these results, *Mycoplasma genitalium* permease protein (SEQ ID NO: 1) represents a promising candidate to initiate an anti-HIV vaccine protocol based on its ability to prime B-cell clones, able to further recognize native HIV-antigens.

The invention is as defined in the appended set of claims.

Therefore, a first aspect of the invention relates to a *Mycoplasma genitalium.* permease antigen or a polynucleotide encoding said antigen in expressible form, for use in the prevention and/or treatment of HIV infection, wherein said permease antigen comprises the amino acid sequence SEQ ID NO: 4 or an amino acid sequence which is at least 95 % identical to SEQ ID NO: 4 and which comprises an epitope cross-reactive with HIV-gp41 ectodomain epitope, and wherein said antigen induces HIV cross-reactive and HIV neutralizing anti-permease antibodies. In the following description, unless otherwise specified, *Mycoplasma* permease, , *M.* permease or permease refers to *M. genitalium* permease protein of SEQ ID NO: 1.. *Mycoplasma genitalium* permease is the product of the locus tag MG 468 (complement of positions 570994 to 576345 on *Mycoplasma genitalium* G37 genome sequence GenBank accession number L43967.2). *M. genitalium* permease amino acid sequence corresponds to GenBank accession number AAC72488.1 or SEQ ID NO: 1.

The amino acid sequence SEQ ID NO: 4 corresponds to a truncated permease protein (residues 431 to 875 of *M. genitalium* permease amino acid sequence SEQ ID NO: 1) including HIV cross-reactive neutralizing epitopes from the ectodomain and C-terminal tail of HIV gp41.

In the following description, the standard one letter amino acid code is used.

The *Mycoplasma* permease antigen or permease antigen for use according to the invention refers to an antigen comprising a protein derived from *M*. *genitalium* permease of SEQ ID NO: 1, which protein comprises the amino acid sequence SEQ ID NO: 4 or an amino acid sequence which is at least 95 % identical to SEQ ID NO: 4 and is capable of inducing anti-permease antibodies which cross-react with HIV and neutralize HIV. The antibodies induced by the permease antigen which cross-react with HIV and neutralize HIV are named hereafter as anti-permease antibodies, HIV cross-reactive and neutralizing or HIV cross-reactive and neutralizing anti-permease antibodies.

In particular, the permease antigen for use of the invention is capable of priming B-cell clones able to further recognize native HIV-antigens.

The permease antigen for use of the invention may be isolated, natural, recombinant or synthetic antigen comprising or consisting of *Mycoplasma genitalium* permease protein of SEQ ID NO: 1 or immunogenic derivative thereof such as for example *M. genitalium* permease variant, and modified protein thereof. *M. genitalium* permease protein variants are derived from wild-type amino acid sequences by the introduction of one or more mutations (deletion, insertion, and/or substitution) at specific amino acid positions. *M. genitalium* permease protein variants include natural variants resulting from *M. genitalium* permease gene polymorphism as well as artificial variants. The antigens derived from *M. genitalium* permease protein for use of the invention are functional antigens, meaning that they are capable of inducing anti-permease antibodies, HIV cross-reactive and HIV infection neutralizing.

The HIV cross-reactive and neutralizing antibodies which are induced by the permease antigen for use of the invention recognize related epitopes in *M. genitalium* permease and HIV Env proteins.

The permease antigen for use of the invention which is capable of inducing a broadly neutralizing protective immune response against HIV infection is useful as preventive and/or therapeutic HIV vaccine.

The properties of the permease protein antigen for use of the invention can be readily verified by technique known to those skilled in the art such as those described in the examples of the present application.

The polynucleotide encoding the antigen in expressible form refers to a nucleic acid molecule which, upon expression in a cell or a cell-free system results in a functional antigen. The polynucleotide may be an isolated polynucleotide encoding said antigen, such as the natural polynucleotide of *Mycoplasma genitalium* encoding said antigen.

The permease antigen for use of the invention, which is capable of inducing anti-permease antibodies cross-reactive with HIV and neutralizing HIV, comprises at least one B cell or antibody epitope from *M. genitalium* permease which is a HIV cross-reactive and neutralizing epitope.

The permease epitope cross-reacts preferably with an HIV-Env epitope, more preferably a HIV-gp41 epitope.

The permease epitope may advantageously cross-react with a HIV-gp41 ectodomain epitope from SEQ ID NO: 2.

Alternatively, the permease epitope may advantageously cross-react with an epitope of HIV-gp41 C-terminal tail. The permease epitope may cross-react with the amino acid sequence: LVGLRIVFAVLSIVNRVRQGYSPLSFQTHLPTPRGP (SEQ ID NO: 3) from HIV-gp41 C-terminal tail (positions 699 to 734 of HIV gp41, according to the numbering system of Ratner et al., Nature, 1985, 313, 277-284), which exhibits sequence homology with *M. genitalium* permease of SEQ ID NO: 1, as demonstrated in the examples of the present application.

The permease antigen for use the invention comprises one or more HIV cross-reactive and neutralizing epitope(s), wherein each epitope may be either linear or conformational. In some aspects, the permease antigen comprises at least two identical or different HIV cross-reactive and neutralizing epitopes from *M. genitalium* permease linked to each other directly or via a peptidic spacer arm.

The percent amino acid sequence identity is defined as the percent of amino acid residues in a Compared Sequence that are identical to the Reference Sequence after aligning the sequences and introducing gaps if necessary, to achieve the maximum sequence identity. The Percent identity is then determined according to the following formula: Percent identity = 100 x [1- (C/R)], wherein C is the number of differences between the Reference Sequence and the Compared sequence over the entire length of the Reference sequence, wherein (i) each amino acid in the Reference Sequence that does not have a corresponding aligned amino acid in the Compared Sequence, (ii) each gap in the Reference Sequence, and (iii) each aligned amino acid in the Reference Sequence that is different from an amino acid in the Compared Sequence constitutes a difference; and R is the number amino acids in the Reference Sequence over the length of the alignment with the Compared Sequence with any gap created in the Reference Sequence also being counted as an amino acid.

Alignment for purposes of determining percent amino acid sequence identity can be achieved in various ways known to a person of skill in the art, for instance using publicly available computer software such as BLAST (Altschul et al., J. Mol. Biol., 1990, 215, 403-). When using such software, the default parameters, e.g., for gap penalty and extension penalty, are preferably used. For amino acid sequences, the BLASTP program uses as default a word length (W) of 3 and an expectation (E) of 10.

The sequence of the permease antigen for use of the invention is preferably selected from the group consisting of SED ID NO: 4 and a sequence which differs from SEQ ID NO: 4 by dispersed deletions and/or insertions of one to five amino acids in said sequence, amino acid substitutions, and/or N-terminal deletion(s) of one or more amino acids in said sequence. The amino acid substitution(s) in SEQ ID NO:4 are advantageously chosen from conservative substitutions, i.e., substitutions of one amino acid with another which has similar chemical or physical properties (size, charge or polarity), which generally does not adversely affect the antigenic properties of the protein/peptide. More preferably, said conservative substitution(s) are chosen within one of the following five groups: Group 1-small aliphatic, non-polar or slightly polar residues (A, S, T, P, G); Group 2-polar, negatively charged residues and their amides (D, N, E, Q); Group 3-polar, positively charged residues (H, R, K); Group 4-large aliphatic, nonpolar residues (M, L, I, V, C); and Group 5-large, aromatic residues (F, Y, W)..

The permease antigen for use of the invention may be a full length permease protein or an immunogenic fragment thereof. In some embodiments the permease antigen comprises or consists of a protein of less than 500 amino acids.

Examples of preferred permease antigens for use of the invention comprise a protein comprising or consisting of SEQ ID NO: 4 or SEQ ID NO: 7.

The permease protein for use of the invention may comprise or consist of natural amino acids (20 gene-encoded amino acids in a L- and/or D-configuration) linked via a peptide bond as well as peptidomimetics of such protein where the amino acid(s) and/or peptide bond(s) have been replaced by functional analogues. Such functional analogues include all known amino acids other than said 20 gene-encoded amino acids. A non-limitative list of non-coded amino acids is provided in Table 1A of US 2008/0234183. The permease protein for use of the invention may be a modified protein derived from the above proteins by introduction of any chemical modification into one or more amino acid residues, peptide bonds, N-and/or C-terminal ends of the protein, as long as the modified protein is functional. These modifications which are introduced into the protein by the conventional methods known to those skilled in the art, include, in a non-limiting manner: the substitution of a natural amino acid with a non-proteinogenic amino acid (D amino acid or amino acid analog); the modification of the peptide bond, in particular with a bond of the retro or retro-inverso type or a bond different from the peptide bond; the cyclization, and the addition of a chemical group to the side chain or the end(s) of the protein/peptide, in particular for coupling an agent of interest to the protein of the invention. These modifications may be used to increase its antigenicity, immunogenicity and/or bioavailability, or to label the protein/peptide. For example, the permease antigen may be conjugated to a GPI moiety for anchoring the antigen to the cell membrane and exposing said antigen at the cell-surface.

In another aspect, the permease antigen for use of the invention is a fusion or chimeric protein comprising an amino acid sequence fused to the N-terminal and/or C-terminal end(s) of a permease protein comprising the amino acid sequence SEQ ID NO: 4 or an amino acid sequence which is at least 95 % identical to SEQ ID NO: 4 as defined above. The length of the chimeric protein is not critical to the invention as long as the permease antigen is functional. The permease protein is fused to one or more other protein/peptide moieties including other antigen(s), in particular HIV antigens, and/or other protein/peptide moieties which enhance B-cell activation, and/or trafficking to the lymph nodes, and/or increase the stability, bioavailability, and/or allow the purification, detection, immobilization, production in expression systems, of the permease antigen of the invention.

These moieties may be selected from: (i) a protein component of a virus-like particle for exposing the antigen of the invention at the surface of virus-like nanoparticles, (ii) a membrane anchoring moiety such as the transmembrane domain of a transmembrane protein for anchoring the antigen at the cell-surface and exposing said antigen at the cell-surface (iii) a labeling moiety such as a fluorescent protein (GFP and its derivatives, BFP and YFP), (iv) a reporter moiety such as an enzyme tag (luciferase, alkaline phosphatase, glutathione-S-transferase (GST), β-galactosidase), (v) a binding moiety such as an epitope tag (polyHis6, FLAG, HA, myc.), a DNA-binding domain, a hormone-binding domain, a poly-lysine tag for immobilization onto a support, (vi) a stabilization moiety, and (vii) a targeting moiety for addressing the chimeric protein to a specific cell type or cell compartment. In addition, the permease antigen may be separated from the peptide/protein moiety by a linker which is long enough to avoid inhibiting interactions between the permease antigen peptide and the protein/peptide moiety. The linker may also comprise a recognition site for a protease, for example, for removing affinity tags and stabilization moieties from the purified chimeric protein according to the present disclosure.

The polynucleotide encoding the protein in expressible form is synthetic or recombinant DNA, RNA or combination thereof, either single- and/or double-stranded. Preferably the polynucleotide comprises a coding sequence which is optimized for the host in which the protein/peptide is expressed.

In another preferred embodiment, the polynucleotide comprises or consists of SEQ ID NO: 5 or 6.

In another aspect, the polynucleotide for use of the invention is inserted in a vector. Preferably, said recombinant vector is an expression vector capable of expressing said polynucleotide when transfected or transformed into a host cell such as a prokaryotic or eukaryotic cell. The polynucleotide is inserted into the expression vector in proper orientation and correct reading frame for expression. Preferably, the polynucleotide is operably linked to at least one transcriptional regulatory sequence and, optionally to at least one translational regulatory sequence. Recombinant vectors include usual vectors used in genetic engineering, vaccines and gene therapy including for example plasmids and viral vectors.

In some embodiments, the permease antigen or the polynucleotide encoding said antigen are included in an immunogenic or vaccine composition, comprising a pharmaceutically acceptable vehicle, and optionally, at least one pharmaceutically acceptable carrier and/or adjuvant.

The immunogenic or vaccine composition for use according to the invention is capable of inducing a functional mucosal protective immune response against HIV infection which can be measured by various assays which are known to those skilled in the art. Examples of such assays include with no limitation: HIV virus capture assays, Antibody-Dependent-Cellular-Cytotoxicity (ADCC) assays using primary CD89+-polynuclear effector cells, Fc-mediated inhibition assays on macrophages, macrophage phagocytosis induction assays, viral replication inhibition assays, HIV infection neutralizing assays, virus aggregation assays and transcytosis inhibition assays.

In some aspects, the permease antigen for use of the invention is an isolated antigen. In other aspects the permease antigen for use of the invention comprises whole-cell live, attenuated or inactivated *M. genitalium.*

The polynucleotide encoding said antigen is preferably inserted in an expression vector.

The composition for use of the invention comprises a therapeutically effective dose of the antigen/polynucleotide/vector, *e.g*., sufficient to induce a specific protective immune response against HIV, in the individual to whom it is administered. The effective dose is determined and adjusted depending on factors such as the composition used, the route of administration, the physical characteristics of the individual under consideration such as sex, age and weight, concurrent medication, and other factors, that those skilled in the medical arts will recognize.

The composition is generally administered according to known procedures used for vaccination, at dosages and for periods of time effective to induce a specific protective immune response against HIV. The administration may be by injection or by oral, sublingual, intranasal, rectal or vaginal administration, inhalation, or transdermal application. Preferably, the administration is by intradermal injection or intranasal, intravaginal or intrarectal administration.

The pharmaceutical vehicles are those appropriate to the planned route of administration, which are well known in the art.

The carrier may advantageously be selected from the group consisting of: uni- or multi-lamellar liposomes, ISCOMS, virosomes, viral pseudo-particules, saponin micelles, saccharid (poly(lactide-co-glycolide)) or gold microspheres, and nanoparticules. Preferably, the composition comprises liposomes.

The adjuvant may advantageously be selected from the group consisting of: polyI:C (polyinosine-polycytidylic acid), oil emulsion, mineral substances, bacterial extracts, CpG-containing oligonucleotide, saponin, alumina hydroxide, monophosphoryl-lipid A (MPLA, a TLR4 agonist) and squalene.. Preferably, the adjuvant is capable of inducing an IgA antibody response, such as for example, polyI:C (polyinosine-polycytidylic acid) adjuvant. More preferred adjuvants include polyI:C and MPLA. The composition is formulated for administration by a number of routes. Preferably, the composition is formulated for intradermal injection or local application, more preferably for intradermal, intranasal, intravaginal, or intrarectal administration. For example, the composition is formulated in a gel for local application.

According to a preferred embodiment of the composition for use of the invention, said composition further comprises an antibody directed to said permease antigen; the antibody forms an antigen-antibody complex with the antigen which has the advantage of increasing the immunogenicity of the antigen. Preferably, the antibody is an IgA, more preferably a human or humanized IgA. The antibody is advantageously a monoclonal antibody. For example, the antibody is a human chimeric IgA, such as the human chimeric IgA monoclonal antibody C3G4 comprising a murine IgVH segment of SEQ ID NO: 8, a murine IgVL segment of SEQ ID NO: 9, a human Ig-alpha1 constant segment of SEQ ID NO: 10 and a murine Ig-kappa constant segment of SEQ ID NO: 11, which is disclosed in the examples of the present application.

A preferred vaccine composition for use of the invention, comprises a *M.genitalium* permease antigen formulated in liposomes containing monophosphoryl lipid A (MPLA). Both liposomes and MPLA contribute as adjuvant. One mL of liposomal suspension may contain 1 mg of *M. genitalium* permease antigen and 0.800 mg of MPLA. The vaccine composition may be formulated as a gel, for local route administration, for example nasal or vaginal route. The gel may comprise 4 % w/w Natrosol® 250 HHX (Hydroxyethyl cellulose) and 1.1% w/w Benzyl alcohol, in PBS.

The permease antigen or polynucleotide encoding said antigen as defined above is a prophylactic and/or therapeutic vaccine, for use in the prevention and/or treatment of HIV infection by administration to an individual of a therapeutically effective amount of the permease antigen, polynucleotide and/or vector as described above, preferably included in an immunogenic or vaccine composition as described above.

The administration of the permease antigen is used to prime B-cell clones, able to further recognize native HIV-antigens. Following, the initial priming, the anti-HIV immune response may be boosted by administration of HIV antigen(s), in particular HIV-Env antigen(s). A mixture of different HIV antigens may be used. Preferred HIV antigens are HIV gp41 ectodomain and/or C-terminal tail antigens.

The vaccination protocol may comprise an initial priming with the permease antigen comprising three administrations of permease antigen at one month interval and a final boost with HIV-1 antigen(s), one month after the last administration of permease antigen.

Herein disclosed is a combined preparation containing a permease antigen, polynucleotide, vector as described herein and HIV antigen(s), in particular HIV-Env antigen(s), for the simultaneous, separate or sequential use in the prevention and/or treatment of HIV infection, in particular HIV-1 infection.

Another aspect of the invention relates to an isolated anti-permease antibody which is HIV cross-reactive and neutralizing, or a functional fragment of said antibody comprising at least the antibody binding site, wherein the antibody is directed against a *M. genitalium* permease antigen which comprises the amino acid sequence of SEQ ID NO: 4 or an amino acid sequence which is at least 95 % identical to SEQ ID NO: 4 and which comprises an epitope cross-reactive with a HIV-gp41 ectodomain epitope. Preferably, the antibody is an IgA, more preferably a human or humanized IgA, such as a human chimeric IgA. The antibody is advantageously a monoclonal antibody. In a preferred embodiment, the antibody is human chimeric IgA monoclonal antibody produced by immunisation of an HAMIGA transgenic mouse (EP patent 1 680 449) with a permease antigen as described above. For example, the antibody is the human chimeric IgA monoclonal antibody C3G4 comprising a murine IgVH segment of SEQ ID NO: 8, a murine IgVL segment of SEQ ID NO: 9, a human Ig-alpha1 constant segment of SEQ ID NO: 10 and a murine Ig-kappa constant segment of SEQ ID NO: 11, which is disclosed in the examples of the present application.

Herein disclosed is an isolated polynucleotide encoding said HIV cross-reactive and neutralizing anti-permease antibody or functional fragment thereof. The polynucleotide may comprise the sequence SEQ ID NO: 12 or 13 which encodes the IgVH segment of SEQ ID NO: 8 and the IgVL segment of SEQ ID NO: 9, respectively.

The anti-permease antibody is a useful component of the immunogenic or vaccine composition as described above, it is also a companion diagnostic tool to evaluate the protective immune status evolution and maturation of patients treated or immunized against HIV infection, a predictive marker of resistance to HIV infection and a tool for designing HIV immunogens capable of inducing a protective neutralizing antibody response against HIV infection.

Herein disclosed is a method for the identification of a new vaccine candidate antigen against a pathogen of interest, comprising:
a) identifying an antigen in the mucosal microbiota of an individual naturally resistant to said pathogen, in particular a human individual, wherein the microbiotal antigen is from a microorganism different from said pathogen,
b) generating an antibody against said microbiotal antigen in a),
c) assaying the neutralizing activity of the antibody obtained in b) against said pathogen, wherein a neutralizing activity of the antibody against said pathogen indicates that the microbiotal antigen is a new vaccine candidate antigen against said pathogen, for an individual which is susceptible to said pathogen.

The natural microbiota may be from the vaginal, naso-pharyngeal, lung or gastro-intestinal mucosa.

The pathogen is a microorganism which may be pathogenic in some individuals but not pathogenic in other individuals which differ from the first ones by some factors such as for example age, sex, medical history, immune status and microbiota.

The pathogen may be a virus. In some aspects of said method, said pathogen is HIV and/or said microbiota is from the vaginal mucosa. The antibody is advantageously generated by immunization of an HAMIGA transgenic mouse (EP patent 1 680 449) with the microbiotal antigen, to produce human chimeric IgAs directed against said antigen.

A neutralizing activity is indicative that the microbiotal antigen is capable of inducing a protective immune response against said pathogen. The neutralization activity may be related to an antibody which recognizes epitopes which may not be identified by standard binding assays.

The polynucleotide for use according to the invention is prepared by the conventional methods known in the art. For example, it is produced by amplification of a nucleic sequence by PCR or RT-PCR, by screening genomic DNA libraries by hybridization with a homologous probe, or else by total or partial chemical synthesis. The recombinant vectors are constructed and introduced into host cells by the conventional recombinant DNA and genetic engineering techniques, which are known in the art.

The protein for use according to the invention is prepared by the conventional techniques known to those skilled in the art, in particular by expression of a recombinant DNA in a suitable cell system (eukaryotic or prokaryotic) or by solid-phase or liquid-phase synthesis. More specifically, the protein and its derivatives are usually produced from the corresponding cDNA, obtained by any means known to those skilled in the art; the cDNA is cloned into an eukaryotic or prokaryotic expression vector and the protein produced in the cells modified with the recombinant vector is purified by any suitable means, in particular by affinity chromatography. The peptide and its derivatives are usually solid-phase synthesized, according to the Fmoc technique, originally described by Merrifield et al. (J. Am. Chem. Soc., 1964, 85: 2149-) and purified by reverse-phase high performance liquid chromatography.

The practice of the present invention will employ, unless otherwise indicated, conventional techniques which are within the skill of the art. Such techniques are explained fully in the literature. In addition to the above arrangements, the invention also comprises other arrangements, which will emerge from the description which follows, which refers to exemplary embodiments of the subject of the present invention, with reference to the attached drawings in which:

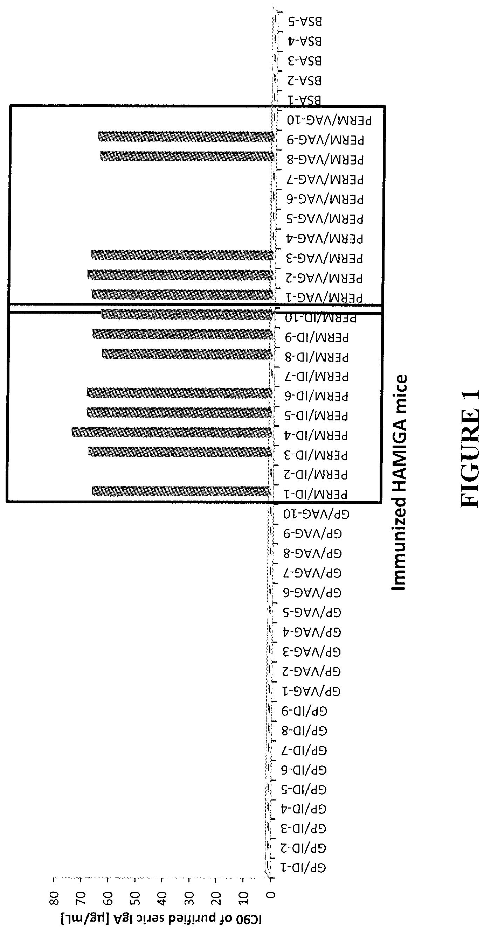

- figure 1 illustrates HIV-1 infection neutralization potency (IC90) in PBMC-based neutralizing assay of purified seric IgA from HAMIGA mice immunized with *Mycoplasma genitalium* permease antigen (PERM) or HIV gp41-ectodomain polypeptide (GP). HAMIGA mice (10 mice per group) were immunized via two different routes (vaginal route (VAG) or intradermal route (ID)) and with two different antigens, a recombinant truncated permease antigen from *M.genitalium* (PERM) or the HIV gp41-ectodomain polypeptide (GP). Control HAMIGA mice (5 mice per group) were immunized with Bovine Serum Albumin (BSA) by the ID route. Seric IgA were collected and purified by affinity chromatography. Neutralizing activity of seric IgA from each immunized mouse was measured against SF162 virus strain in a PBMC based-neutralizing assay. The neutralizing activity is expressed as IC90 (Inhibitory concentration 90 %), *i.e*., the concentration (µg/mL) of purified seric IgA that caused a 90 % reduction of HIV virus infection. Only seric IgAs from permease immunized mice were able to exhibit an IC90 potency of HIV-1 infection neutralization at the range of 60 µg/mL. Despite a strong anti-gp41 binding, none of the seric IgAs from gp41-ectodomain polypeptide immunized mice was able to generate an IC90 neutralizing immune response.

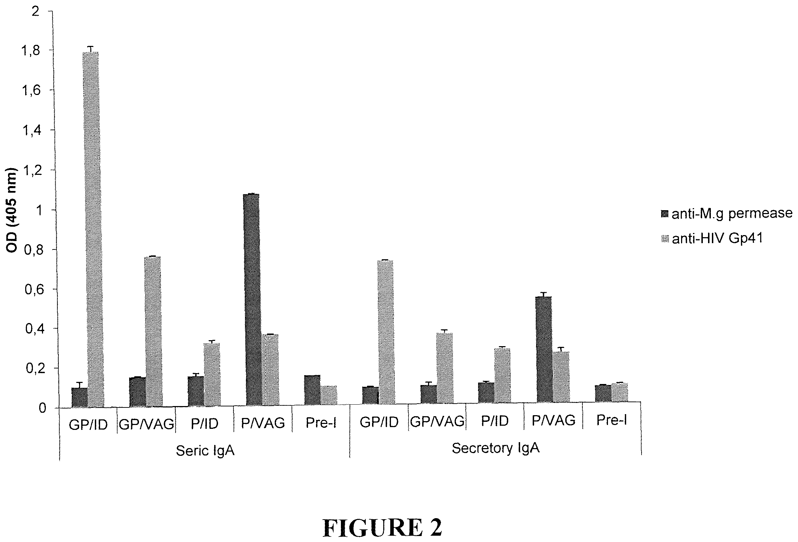

- -figure 2 shows cross-reactivity by ELISA of anti-*Mycoplasma genitalium* permease polyclonal seric and secretory IgA antibodies with HIV gp41. HAMIGA transgenic mice were immunized with *Mycoplasma genitalium* (M.g.) permease antigen (P) or gp41-ectodomain polypeptide (GP) by the intradermal (ID) or intra-vaginal (VAG) routes. Anti HIV-1 gp41 and anti-*M.g*. permease specific polyclonal IgA antibodies were analysed by ELISA in serum and vaginal secretions of Pre-immune mice (Pre-I) and immunized mice. Although polyclonal IgA (seric and vaginal) from gp41-immunized HAMIGA mice do not show a significant binding on the permease in ELISA, polyclonal IgA from *M.genitalium* permease-immunized HAMIGA mice exhibit a significant cross-reactivity against gp41 ectodomain antigen.

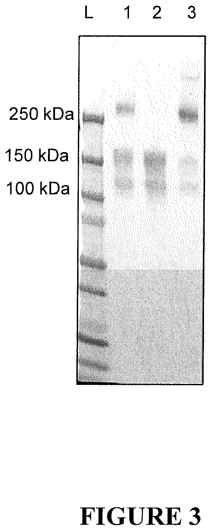

- figure 3 represents Coomassie-blue-stained SDS-PAGE analysis of HIV-1 neutralizing human/murine chimeric monoclonal IgA1_{κ} antibody C3G4. C3G4 was purified by affinity-chromatography and monomeric and polymeric forms of IgA were separated by size-exclusion chromatography. C3G4 contains the two forms - monomeric and polymeric - of a monoclonal IgA1. L: molecular weight marker. 1. Total fraction of mAb C3G4. 2. Monomeric-IgA enriched fraction of mAb C3G4. 3. Polymeric-IgA enriched fraction of mAb C3G4.

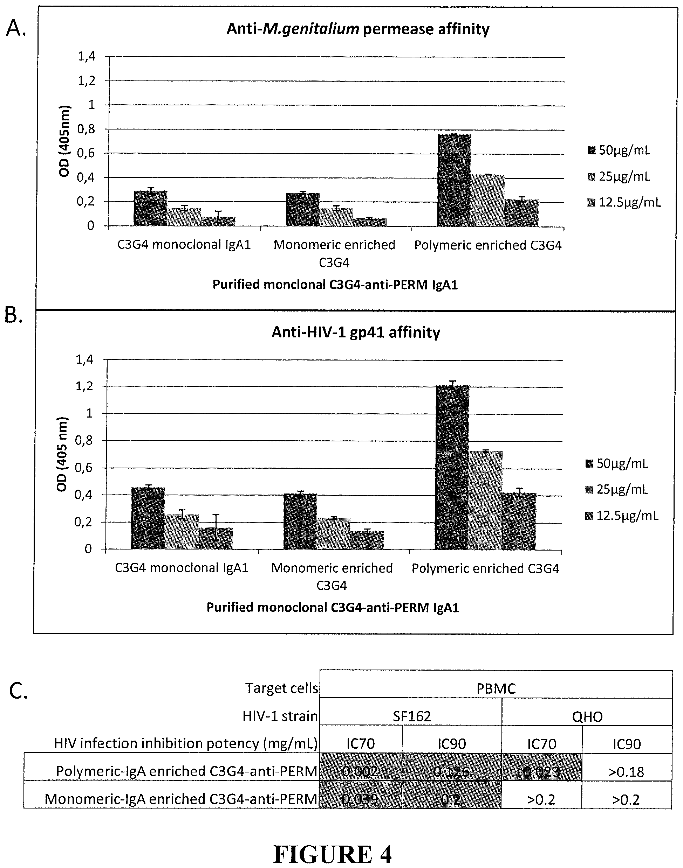

- figure 4 illustrates the binding and neutralizing activity of C3G4 monoclonal anti-*M*. *genitalium* permease IgA1. A. The binding of total IgA, monomeric IgA-enriched and polymeric IgA-enriched fractions of C3G4 to *M. genitalium* permease was analysed by ELISA using truncated *M. genitalium* permease as antigen. B. The binding of total IgA, monomeric IgA-enriched and polymeric IgA-enriched fractions of C3G4 to HIV gp41 was analysed by ELISA using HIV gp41-ectodomain polypeptide as antigen. C. Neutralizing activity of monomeric IgA and polymeric IgA enriched fractions of C3G4 was measured against HIV-1/SF162 and HIV-1/QHO virus strains in a PBMC based-neutralizing assay. The neutralizing activity is expressed as IC70 (Inhibitory concentration 70 %) and IC90 (Inhibitory concentration 90 %), *i.e*., the concentration (mg/mL) of monomeric enriched or polymeric enriched fraction of C3G4 that caused a 70 % and 90 % reduction of HIV virus infection, respectively.

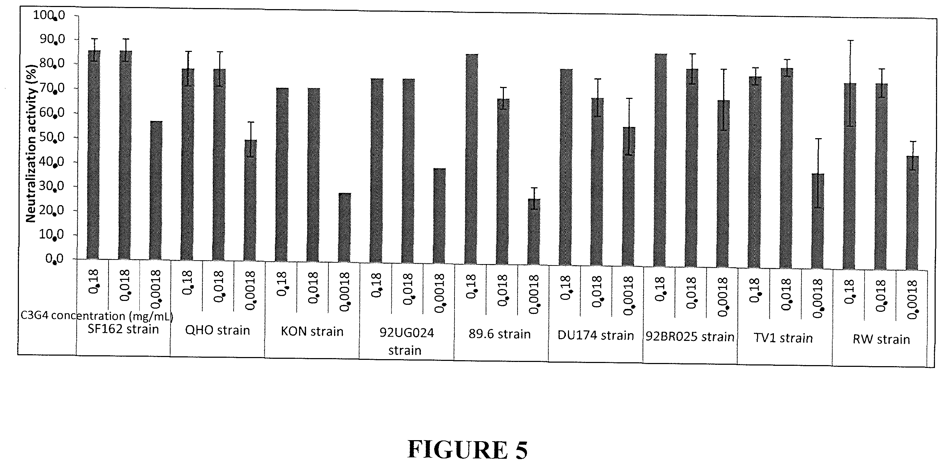

- figure 5 illustrates the neutralization activity of C3G4 monoclonal anti-*M*. *genitalium* permease IgAlin PBMC-based neutralizing assay on nine HIV-1 strains. Neutralizing activity of purified C3G4 polymeric IgA1 was tested on different HIV-1 laboratory and primary strains. Purified C3G4 polymeric IgA1 exhibited a neutralization activity on nine different HIV-1 strains with an IC80 < 18 µg/mL against HIV-1_{SF162}, HIV-1_{92BR025} and HIV-1_{TV1} isolates, an IC50 <1.8 µg/mL against HIV-1_{QHO} and HIV-1_{DU174} isolates and an IC50 <18 µg/mL against HIV-1_{KON}, HIV-1_{92UG024}, HIV-1_{89.6} and HIV-1_{RW} isolates.

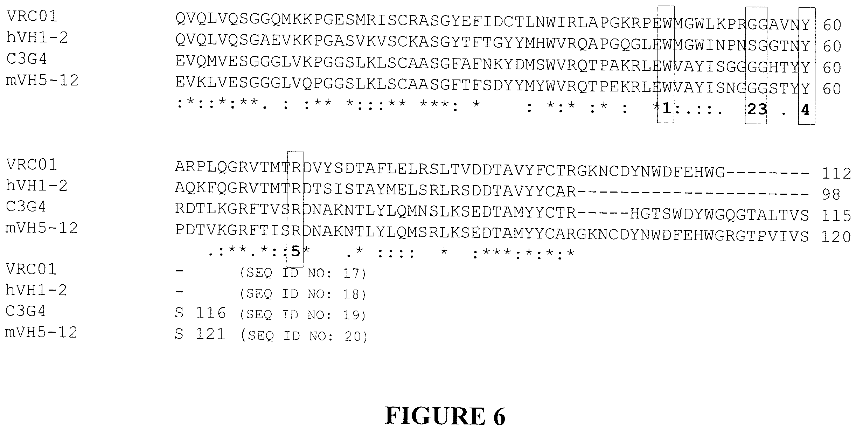

- figure 6 represents the alignment of IgH variable segments of HIV neutralizing human mAb VRC01 (SEQ ID NO: 17) directed to CD4bs-gp120 and its human unmutated ancestor IgVH1-2^{∗}2 (SEQ ID NO: 18); HIV-1 neutralizing human/murine chimeric monoclonal IgA1_{κ} antibody C3G4 (SEQ ID NO: 19) and its murine unmutated ancestor IgVH5-12 (SEQ ID NO: 20).

### Example 1: Identification of the vaginal immune response in uninfected women highly exposed to HIV

### 1. Material and methods

### 1.1 Cohorts

A cohort of 32 HIV highly-exposed uninfected women (HEUW) from sero-discordant couples was investigated in collaboration with the GHESKIO Centers (Haitian Group for the Study of Kaposi's Sarcoma and Opportunistic Infections) in Haiti. The control cohort included 15 HIV non-exposed uninfected subjects. None of the subjects had CCR5-Delta 32 homozygous genotype and two were heterozygous, one in HEUW group, the other in control group. The study was approved by ethical committee and informed consent was obtained from all women prior sampling.

### 1.2 ELISA on vaginal secretions

Vaginal secretions of HEUW and controls were assayed for HIV gp41-specific IgG1 and IgA antibody responses by ELISA, as previously described (Hocini et al., AIDS Res. Hum. Retroviruses, 1997, 13, 1179-).

### 1.3 Western-blot on vaginal secretions

Vaginal secretions of HEUW and controls were assayed for HIV gp160, gp110, gp41, p68, p55, p53, p40, p34 and p25 specific IgG1 and IgA antibodies by Western-Blot as previously described (Hocini et al., AIDS Res. Hum. Retroviruses, 1997, 13, 1179-).

### 1.4 Inhibition of transcytosis assay

Inhibition of transcytosis of assay was performed as previously described (Bélec, L. et al. The Journal of infectious diseases, 2001, 184, 1412-1422).

### 1.5 Cloning of mucosal cells IgG1 and IgA variable regions into phagemid vector

Separate libraries of IgA and IgG1 Fab fragments were constructed. Briefly, messenger RNAs extracted from mucosal cells of two selected subjects were used for separate amplification of IgG1 and IgA variable regions. PCR primers corresponding to the 5' end of all VH and VL variable domain families were combined individually with a primer derived from IgG1 or IgA first constant domain (Persson et al., Proc. Natl. Acad. Sci. U S A., 1991, 88, 2432-6).The amplified DNA fragments corresponding to heavy and light chains were then cloned into a phagemid vector (Persson et al., Proc. Natl. Acad. Sci. U S A., 1991, 88, 2432-6), allowing the surface display of Fab fragment (Barbas et al., Proc. Natl. Acad. Sci. USA, 1991, 88, 7978-82).

### 1.6 Sequencing of recombinant IgG and IgA Fab

Individual clones, from both IgG1 and IgA libraries were sequenced using the standard Sanger dideoxy technique.

### 1.7 Expression of recombinant IgG1 and IgA Fab fragments

Recombinant Fab fragments were expressed using both *E. coli* (Studier and Moffat, J. Mol. Biol., 1986, 189, 113-130) and Cell-free translation (Ryabova et al., Methods Mol. Biol. 1998, 77, 179-93).

### 1.8 Analysis of VH mutations

VH mutations of recombinant IgG1 and IgA were analysed as described in Wang et al., BMC bioinformatics, 2008, 9 Suppl 12, S20.

### 1.9 Identification of antibody epitope by phage displayed random peptide libraries screening

In order to identify the epitope recognized by the antibody, the recombinant antibody Fab fragment was screened against commercial phage displayed random peptide libraries (Ph.D. -12™ Phage Display Peptide Library Kit, NEW ENGLAND BIOLABS), according to the manufacturer's instructions. Typically, 4 to 5 rounds of selection (bio-panning) were applied.

### 1.10 Statistical analysis

A Yates' chi-squared test was used to analyze the samples from Table I. The difference between control group and HIV-highly exposed group is statistically significant as long as *p value*<0.05*.*

### 2. Results

The immune response in the genital tract of a cohort of HIV highly exposed uninfected women (HEUW) from sero-discordant couples was investigated in collaboration with the GHESKIO Centers (Haitian Group for the Study of Kaposi's Sarcoma and Opportunistic Infections) in Haiti. Vaginal fluids from 32 individuals were analyzed by ELISA and Western Blot, and compared with 15 control HIV non-exposed uninfected subjects. None of the subjects had CCR5-Delta 32 homozygous genotype and two were heterozygous, one in HEUW group, the other in control group. Vaginal secretions were tested for specific IgG1 and IgA antibody responses against gp41 in ELISA. ELISA results were confirmed by western blot. Only samples showing a strong positivity with both techniques were retained for further investigation and antibody library construction.

Although all subjects were seronegative for HIV infection, 53 % of the HEUW were positive in vaginal secretions for anti-gp41 IgA versus only 13 % in the control group (p < 0.02) whereas no significant difference was observed for anti-gp41 IgG responses (Table I).

**Table I: HIV-1 specific immune response in vaginal secretions of HEUW by WB and ELISA**

| Antibody | Positives in Control group (n=15) | Positives in HIV highly exposed group (n=32) | P value |
|---|---|---|---|
| IgA WB/gp160 | 0 (0%) | 3 (10%) | NS |
| IgA WB/gp110 | 0 (0%) | 1 (3%) | NS |
| IgA WB/p68 | 0 (0%) | 1 (3%) | NS |
| IgA WB/p55 | 0 (0%) | 2 (6%) | NS |
| IgA WB/p53 | 0 (0%) | 1 (3%) | NS |
| **IgA WB/gp41** | **2 (13%)** | **17 (53%)** | **0.02** |
| IgA WB/p40 | 0 (0%) | 1 (3%) | NS |
| IgA WB/p34 | 0 (0%) | 4 (12%) | NS |
| IgA WBP/p25 | 1 (6%) | 2 (6%) | NS |
| IgA ELISA/gp41 | 3 (20%) | 9 (28%) | NS |
| | | | |
| IgG1WB/gp160 | 0 | 5 (16%) | 0.16 |
| IgG1 WB/gp110 | 0 | 2 (6%) | NS |
| IgG1 WB/p68 | 0 | 1 (3%) | NS |
| IgG1 WB/p55 | 2 (13%) | 3 (10%) | NS |
| IgG1 WB/p53 | 0 | 4 (12%) | NS |
| IgG1 WB/gp41 | 2 | 8 (25%) | NS |
| IgG1 WB/p40 | 0 | 2 (6%) | NS |
| IgG1 WB/p34 | 0 | 1 (3%) | NS |
| IgG1 WB/p25 | 0 | 4 (12%) | NS |
| IgG1 ELISA gp41 | 2 (13%) | 7 (22%) | NS |

| | | | |
|---|---|---|---|
| WB: Western blot ; NS: non- significant | | | |

The functional activity of the secretions was tested by inhibition of transcytosis of HIV_{JRCSF}. None of the secretions were positive in transcytosis functional assays, compared with the broad HIV-1 neutralizing monoclonal antibody 2F5 used as positive control. Therefore the transcytosis test was not further used for screening of samples.

To characterize the antibody response, mucosal cells were collected with a cytobrush from two CCR5 wild type subjects selected on the basis of their strong response against gp41 in ELISA and Western Blot and of long term unprotected sexual relationship with at least one HIV-1 seropositive partner. In order to avoid amplifying genes from the partner, all samples positive for the Prostate Specific Antigen (> 50 ng/mL PSA) were discarded.

After specific amplification, IgG and IgA variable regions were cloned into a phagemid vector allowing the expression of Fab fragments at the surface of filamentous phages. In a first strategy, phages displaying a Fab binding to a recombinant gp41 antigen would have been selected. This screening step was found to be unnecessary due to the observed strongly oligoclonal profile of the cloned repertoire. In a standard situation, the analysis by sequencing of clones randomly picked from the library should give a whole set of different sequences. However, in the case of the selected subjects, the vast majority of clones was identical or very closely similar and represented a large percentage of the total population (up to 75 % for VH chains). This was not due to a bias in amplification or cloning since the same result was obtained when independent libraries were constructed. The same was true for both the IgG1 and IgA libraries. To further characterize this oligoclonal mucosal repertoire, Fab fragments corresponding to the most represented antibodies were expressed as recombinant proteins.

IgG1 (called Toussaint) in association with 2 different light chains, IgA (called Makandal) in association with 2 different light chains, IgG1 (Jacmel) and IgA (Mangue). The most represented light chains were from κ1 and λ4 families. These two light chains were systematically associated with all the heavy chains since the original VH / VL chain pairing was not possible to determine. Analysis of the VH mutations showed that Makandal had a germinal un-mutated profile. In order to identify the recognized epitope, this antibody was screened against commercial phage displayed random peptide libraries. In two independent screenings, two overlapping peptides allowed for the identification of an epitope corresponding to a region in the C-terminal part of gp41: LVGLRIVFAVLSIVNRVRQGYSPLSFQTHLPTPRGP (SEQ ID NO: 3).

It was hypothesized that the mucosal oligoclonal immune response of these women might be responsible for their resistance to HIV infection and that these antibodies were induced in response to a particular microbiota and by cross-reaction with the gp41 domain of HIV envelope, could prevent the viral fusion. In order to see if the sequence of amino acids recognized by Makandal Fab had any homologies with other proteins of the environment, this sequence was compared with a protein sequence databases excluding HIV proteins. Data showed very weak matches, but interestingly, there were consistent matches with bacterial protein sequences. Among these, a permease from *Mycoplasma genitalium* (GenBank accession number AAC72488.1 or SEQ ID NO: 1) was selected.

### Example 2: The permease antigen of M. genitalium is capable of inducing a systemic and a mucosal neutralizing protective antibody response against HIV infection in HAMIGA mice

### 1. Material and methods

### 1.1 Antigens

### Recombinant truncated permease from M. genitalium (rPermease or PERM)

Recombinant truncated permease cDNA (SEQ ID NO: 6) prepared by gene synthesis (GeneART) was inserted in a procaryotic expression vector (pSP401, derived from *pET28 cer* (NOVAGEN); Peubez et al., Microbial Cell factories, 2010, 9, 65-) and expressed in *E.coli* BL21 strain. Tagged by a Histidine tail, the antigen (SEQ ID NO: 7) was purified by affinity chromatography on His-IMAC (BIORAD) column. The purified recombinant permease protein fragment ([rPermease] = 1 mg/mL in Tris 25 mM, Arginine 0.5 M, Sucrose 5 % buffer, pH 8) had a purity of 82 %.

### HIV gp41 ectodomain peptide (GP)

Recombinant HIV-gp41 ectodomain peptide (GP) preparation is disclosed in Krell et al., European Journal of Biochemistry, 2004, 271, 1566-1579. Briefly, a 0.6 kb DNA fragment containing the sequence encoding the ectodomain of gp41 of HIV isolate LAI (amino acid sequence SEQ ID NO: 2, corresponding to amino acid residues 540 to 673 of gp160 (SEQ ID NO: 14)), was obtained by PCR amplification using, as template, a plasmid containing the gp120 sequence and the gp41 sequence of the LAI isolate. The start codon in the forward primer is a naturally occurring methionine residue. A stop codon and the DNA sequence encoding the extension GGGGSHHHHHH (SEQ ID NO: 15) were added to the reverse primer. Platinum HF polymerase (GIBCO INVITROGEN) was used, according to the manufacturer's instructions, for PCR amplification. The PCR amplified fragment was cloned directly into the vector pM1800 using the restriction sites *Nco*I and *Xho*I*.* The expression vector pM1800 is a derivate of pET28c (NOVAGEN, in which the F1 origin of replication has been deleted and replaced with the Cer fragment, allowing for multimer resolution. For protein expression, E. coli BL21(DE3) was transformed with the corresponding plasmids.Tagged by an Histidine tail, the ectodomain antigen (SEQ ID NO: 16) was purified by affinity chromatography on onto a 5 ml Hi-Trap Chelating column (AMERSHAM PHARMACIA BIOTECH). Protein elution was achieved using a buffer comprising 50 mMTris/HCl, 8 M urea, 500 mM NaCl and 500 mM imidazole, pH 8.0. Protein refolding was achieved by dialysis with 50 mM formate, pH 2.8. Protein was then sterile filtered and stored at -45 °C.

### 1.2 Immunization

Immunization was performed in HAMIGA transgenic mice (EP Patent 1 680 449), a transgenic mouse strain producing human/mouse chimeric IgAs with human IgA heavy chain constant regions and mouse light chain constant regions and (heavy and light chain) variable regions. Estrous cycles of HAMIGA transgenic mice were synchronized by treatment with Depoprovera (5 days before the first immunization, 8 days- and 16 days post-immunization- 2 mg/mouse/injection by subcutaneous route). HAMIGA transgenic mice (10 mice per group) were immunized by intradermal (Group ID in ear) or intravaginal (Group VAG) routes twice at two weeks of interval with the recombinant truncated permease of *M.genitalium* (PERM) in polyI:C adjuvant (10 µg/mouse/injection, ratio 1:2.5 PolyI:C (25µg/mouse/injection, INVIVOGEN)). For comparison, ten HAMIGA mice were immunized with gp41-ectodomain polypeptide via the ID route (GP/ID, 10µg/mouse/injection, in polyI:C adjuvant (10 µg/mouse/administration, ratio 1:2.5 PolyIC (25µg/mouse/injection, INVIVOGEN)) and via the vaginal route (GP/VAG, 10µg/mouse/administration, in polyI:C adjuvant (10 µg/mouse/administration, ratio 1:2.5 PolyIC (25µg/mouse/administration, INVIVOGEN)). As a negative control, five HAMIGA mice were immunized with Bovine Serum Albumin (BSA) antigen by ID route.

### 1.3 Vaginal washes harvest

Vaginal cavities of immunized mice were washed each day during a complete estrous cycle (4 days), by gently flushing vaginal cavity with 200 µL of physiological media (NaCl 0.9 %, URGO).

### 1.4 Seric IgA and secretory IgA concentration titration by ELISA

Seric IgA from immunized HAMIGA mice (purified by affinity chromatography) and secretory IgA prepared from vaginal washes were titered by ELISA. Briefly, 96-well plates (Maxisorp®, NUNC) were coated with 1 µg/mL of goat anti-human IgA (BECKMAN COULTER) in PBS buffer overnight at 4°C. Plates were saturated with a BSA 2%/PBS1X buffer during 30 minutes at 37°C. Incubation of the samples (secretory IgA, diluted 10 times in BSA 0.2 %/PBS or purified seric IgA, diluted 100 times in BSA 0.2 %/PBS) was performed at 37 °C during 2 h. Human IgA reference range (from [control hIgA] = 0.2 mg/ml to 1.56 ng/mL) was incubated following the same protocol and revealed by an Alkaline-Phosphatase (AP) labelled-goat anti-hIgA polyclonal antibody (BECKMAN COULTER; diluted 2000 times in BSA 0.2%/BSA).

### 1.5 Preparation of monoclonal IgA

To isolate monoclonal IgA from permease-immunised mice, immortalization of specific B-cell lymphocytes were performed, according to Kölher and Milstein protocol (Kohler, G. & Milstein, C. European Journal of Immunology, 1976, 6, 511-9). Briefly, splenocytes from permease-immunized mice were fused with mice myeloma cells (X63 Sp2/0) and subcloned on 96-wells plates. The supernatants of hybridoma clones were harvested after three weeks of culture and tested for their neutralizing activity on *in vitro* HIV infection inhibition assay. Each clone was cryopreserved in DMSO 10 %/SVF 20 %/DMEM media in liquid nitrogen.

### 1.6 IgA purification

IgA were purified by affinity chromatography. The monomeric and polymeric forms of IgA were separated by size exclusion column chromatography.

### 1.7 Antigen specificity analysis

Permease fragment/gp41-ectodomain specific IgA were assessed by ELISA using Maxisorp® 96-wells plates (NUNC) coated with 1 to 5 µg/mL of antigens overnight at 4°C. Crude supernatants, unpurified IgA from vaginal washes or purified serum IgA (diluted in PBS/Gelatin 0.2 %) were incubated 2 hours at 37°C. Specific IgA binding was revealed with an AP-conjugated goat anti-human IgA antibody (1/2000 diluted, BECKMAN COULTER).

### 1.8 Cell preparation

Blood samples were collected from anonymous healthy donors (Etablissement Français du Sang, EFS). Peripheral Blood Mononuclear cells were obtained by Ficoll-Hypaque sedimentation of Buffy coats. PBMCs were activated with phytohemagglutinin-A (PHA, 1 mg/mL, SIGMA) in RPMI medium-10% fetal calf serum (FCS) supplemented with antibiotics (Penicillin and Streptomycin, 100 Units/mL and 100 µg/mL, respectively) at the concentration of 10.10⁶ cells/mL for 30 minutes. Cells were then cultivated in RPMI medium-10% FCS supplemented with antibiotics at 2.10⁶ cells/mL. After 3 days, cells were frozen. PBMCs from 5 donors were thawed, pooled and culture for one day before being used in the neutralization assay.

TZM-B1 cell line was obtained through the NIH reagent program. TZM-B1 cells were cultured in RPMI medium-10% FCS supplemented with antibiotics (Penicillin and Streptomycin, 100 Units/mL and 100 µg/mL, respectively).

### 1.9. Virus preparation

Primary HIV-1 isolates were amplified on human blood leukocytes, as described previously (Holl et al., Blood, 2006, 107, 4466-4474). Virus stocks collected at peak virus production were concentrated 70-fold with a 100-kDa cutoff polyethersulfone filter (Centricon® Plus-70 Biomax Filter; MILLIPORE). Primary HIV-1 isolates were obtained from the National Institute for Biological Standards and Control (NIBSC): HIV-1_{SF162} isolate (subtype B, R5), HIV-1_{QH0} isolate (subtype B, R5 strain, R5), HIV-1_{89.6} (sub-type B, X4R5), Viruses HIV-1_{DU174} (subtype C, R5), HIV-1_{92BR025} (subtype C R5), HIV-1_{92UG024} (subtype D, X4), HIV-1_{KON} (subtype CRF02-AG, X4). HIV-1 BaL (subtype B) was provided by S. Gartner, M. Popovic, and R. Gallo (NIH).

Pseudoviruses HIV-I_{SF162} and HIV-1_{QH0} (SF162.LS and QH0692.42) were produced by co-transfection of 293T cell line with EnvC3 back bone and Envs from HIV-I_{SF162} and HIV-1_{QH0} respectively.

### 1.10 Neutralization assays

Neutralization assays were performed on human PBMC and TZM-bl cells, as previously described (Mascola et al., J. Virol., 2005, 79, 10103-10107), using two standard reference strains of clade B as Env-pseudotypes viruses (SF162.LS and QH0692.42; Li et al., J. Virol., 2005, 79, 10108-10125). The 50 %, 70%, 80 % or 90 % inhibitory dose was defined as the sample concentration that caused 50 %, 70%, , 80 % or 90 % reduction in relative luminescence units (RLU; Li et al., J. Virol., 2005, 79, 10108-10125) in TZM-bl or percentage of infected cells in PBMC, respectively.

### 1.11 Fc-mediated inhibition of HIV replication in MDMs

Fc-mediated inhibition assay was performed on Monocyte derived macrophages (MDMs). MDMs were generated by culture of CD14+ monocytes with GM-CSF for 5 days. Inhibition of cell free HIV-1 SF162 or HIV-1 BaL replication in MDMs was assessed as previously described by Holl et al. (J. Virol., 2006, 80, 6177-6181; J. Immunol., 2004, 173, 6274-6283) . Briefly, antibodies and virus were incubated for 1 h before addition to MDMs. Virus replication was measured after 48 h by the intracellular staining of p24 in MDMs by flow cytometry. Percentage of infected cells compared to control infected macrophages without antibodies was determined.

### 2. Results

A truncated permease antigen from *M. genitalium* (SEQ ID NO: 7, corresponding to amino acid sequence 431-875 of *M. genitalium* permease sequence SEQ ID NO: 1) was expressed in *E. coli* and used to immunize HAMIGA transgenic mice expressing chimeric human IgA. Groups of ten HAMIGA mice received two administrations of permease fragment in Poly I-C adjuvant via the intradermal (Group PERM/ID) and vaginal routes (Group PERM/VAG). At the same time, groups of ten HAMIGA mice were immunized with gp41-ectodomain polypeptide (SEQ ID NO: 16) (Group GP/ID; Group GP/VAG). As a negative control, five HAMIGA mice were immunized with BSA antigen (Group BSA) by ID route. IgA from vaginal washes and purified serum IgA from immunized and control HAMIGA mice were analysed.

To identify the immunization route and the antigen able to induce neutralizing immune response, neutralizing activity of seric IgA from each immunized mouse was measured against SF162 virus strain, a tier 1 isolate, in a PBMC-based neutralizing assay (figure 1). Although all seric IgA of PERM-immunized mice show ability to cross-react with HIV gp41 epitopes in ELISA (figure 2), only eight seric IgA from PERM/ID immunized mice and five seric IgA from PERM/VAG immunized mice were able to exhibit a strong potency of HIV-1 infection neutralization with an IC90 in a range of 60µg/mL (figure 1). Despite a strong anti-gp41 binding (figure 2), no seric IgA from GP/ID and GP/VAG immunized mice groups was able to reach IC90 neutralizing activity (figure 1). As expected, in Bovine Serum Albumin (BSA)-immunized mice group, purified seric IgAs showed no gp41 binding by ELISA nor HIV-1 infection neutralization activity (figure 1).

In addition, only vaginal IgA pools from PERM/ID and PERM/VAG immunized group (n=10) exhibited a strong neutralization activity against HIV-1 _{SF162} (a tier 1 isolate,) with the lowest IC90 (in a range of 1.5 to 2.5µg/mL; Table II). Vaginal IgA pools from PERM/VAG immunized group (n=10) reached an IC50<2.5µg/mL against QHO isolate (a tier 2 isolate) when performed in a TZM-bl cells based infection inhibition assay.

**Table II: HIV-1 specific neutralizing activity of purified vaginal IgA pools from immunized HAMIGA mice**

| Target cells | | PBMC | | TZM | |
|---|---|---|---|---|---|
| Percentage of inhibition of HIV-1 infection | | **IC90** | | **IC50** | |
| HIV-1 virus | | SF162 | QHO | SF162 | QHO |
| Group of immunized HAMIGA (n=10) | | | | | |
| Vaginal IgA | GP/ID | 0.010 | >0.010 | >0.01 | >0.01 |
| Vaginal IgA | GP/VAG | >0.016 | >0.016 | 0.016 | 0.016 |
| **Vaginal IgA** | **PERM/ID** | **0.0015** | >0.0015 | >0.0015 | >0.0015 |
| **Vaginal IgA** | **PERM/VAG** | **0.0025** | >0.0025 | **0.002** | **0.0025** |
| Vaginal IgA | Pre-immune | >0.0035 | >0.0035 | >0.0035 | >0.0035 |
| Vaginal IgA | BSA | 0.0115 | >0.0115 | >0.0115 | >0.0115 |

| | | | | | |
|---|---|---|---|---|---|
| *HIV-Infection Inhibition titers are expressed in mg/mL | | | | | |

The supernatants of hybridoma clones from permease-immunised mice were tested for neutralizing activity in a PBMC assay against the HIV-1_{SF162} isolate. Out of 900 hybridoma supernatants, 141 showed 80 % neutralisation of the viral infection including 34 that showed 90 % neutralization potency. One clone, C3G4, was sub-cloned three times and anti-permease specificities were confirmed by ELISA. One clone named C3-G4.EA5.IH2.JA11 was finally stabilized. The variable segments of the neutralizing immunoglobulin, a human chimeric IgA1,_{κ}, were sequenced and analysed on IMGT data base (**www.imgt.org/vquest**).

**Table III: Sequence and analysis of the variable segments of the HIV-1 neutralizing human chimeric monoclonal IgA1,_{κ}, C3G4**

| | | | | | |
|---|---|---|---|---|---|
| **Heavy chain** | | | | | |
| | | *IgH-V* | *IgH-D* | | *IgH-J* |
| **mab hIgA1 (C3G4 clone)** | | 5-12-1*01F | 1-1*01 | | 2*01 F |
| **Mouse germline gene Identity** | | 93.06% | | | 82.98 % |
| **AA *Ig*HV sequence** | | | | | |
| | | | | | |
| **Light chain** | | | | | |
| | | *IgH-V* | *IgH-J* | | |
| **mab hIgA1 (C3G4 clone)** | | 15-103*01 | 1*01 F | | |
| **Mouse germline gene Identity** | | 95.34% | 94.74% | | |
| **AA *IgLV* sequence** | | | | | |

The supernatant of C3G4 contains both a monomeric and polymeric monoclonal IgA1 (Figure 3, line 1). Fractions enriched in monomeric (Figure 3, line 2) and polymeric forms of IgA (Figure 3, line 3) were prepared and their affinity for *M.genitalium* permease (figure 4, A) and cross-reactivity with HIV gp41 were tested in ELISA (figure 4, B). In each ELISA test, polymeric-IgA enriched form of C3G4 exhibited the highest affinity for *M.genitalium* truncated permease and gp41-ectodomain polypeptide.

Monomeric IgA and polymeric IgA enriched fractions of purified C3G4 were primarily tested in a PBMC neutralizing assay with two HIV-1 virus isolates, SF162 and QHO (figure 4C). Polymeric IgA enriched form strongly inhibited infection by both HIV-1 isolates SF162 (IC70 of 2µg/mL) and QHO (IC70 of 23µg/mL). 90% inhibition of HIV-I_{SF162} isolate infection was achieved with 126 µg/mL of polymeric IgA enriched C3G4. In each test, polymeric form of C3G4 IgA showed a greater neutralizing potency than the monomeric IgA enriched form of C3G4.

Monomeric and polymeric forms of C3G4 were also tested in a macrophage-based neutralization assay. The assay shows the Fc-mediated inhibitory activity of the antibodies. As shown in Table IV, both forms presented 80 % inhibition potency in PBMC and Macrophages-based assays, in presence of 6µg/mL and 14 µg/mL of C3G4 IgA, respectively. Ninety percent inhibition of HIV-1 infection was achieved when both tests were performed with 58 µg/mL of C3G4 IgA polymeric form. Polymeric form of C3G4 IgA showed a greater neutralizing potency than monomeric form. The higher avidity of polymeric form may allow the antibody to recognize and capture several viral particles, as opposed to the monomeric form.

**Table IV: HIV-1 specific neutralizing activity of monomeric or polymeric enriched fractions of C3G4 IgA antibody**

| Human cell type | | PBMC | | Macrophages | |
|---|---|---|---|---|---|
| HIV-1 isolate | | SF162 | | BaL | |
| HIV-1 infection inhibition titer (in mg/mL) | | IC80 | IC90 | IC80 | IC90 |
| Samples | C3G4-purified IgA1 (containing two forms mix) | >0.125 | >0.125 | **0.031** | **0.200** |
| | C3G4-enriched monomeric IgA1 | **0.094** | >0.094 | **0.023** | **0.094** |
| | C3G4-enriched polymeric IgA1 | **0.006** | **0.058** | **0.014** | **0.058** |

Neutralizing activity of monoclonal C3G4 IgA1 was also tested on different primary HIV-1 isolates (Figure 5). Purified polymeric IgA1 exhibited a neutralization activity on nine different HIV-1 primary isolates at a low IC50 and IC80. C3G4 neutralized HIV-1_{SF162}, HIV-1_{BR025} and HIV-1_{TV1} isolates with a great potency (IC80 comprised in the range of 2 to 20 µg/mL). C3G4 neutralized HIV-1_{QHO}, _{KON}, _{92UG024}, _{89.6} and _{RW} with moderate potency (IC80 > 20 µg/mL).

These data demonstrate that *Mycoplasma genitalium* permease antigen represents a HIV-1 vaccine candidate able to prime a mucosal and systemic broadly-neutralizing protective antibody response against HIV infection in the immunized individuals.

### Discussion

In this study, using HAMIGA mice to produce IgA, immunization with *M. genitalium* permease adjuvanted with polyI:C administered via the ID or vaginal routes elicited specific IgA Abs able to recognize the HIV-Gp41 and to neutralize HIV-1 primary isolates.

IgA is the most abundant antibody isotype produced weekly and delivered by secretion in mucosal area. Because HIV infection is usually initiated at mucosal site of entrance, the inventors assumed that IgA would better mimic the response in mucosa, including the genital mucosa. Pivotal role of polymeric form of IgA is to prevent pathogen entry by immune exclusion, but also polymeric IgA are assumed to agglutinate the virus and block transcytosis (Chomont, N. et al. Virology, 2008, 370, 246-254) and with more efficacy than their IgG homologue (Hur, E. M. et al. Blood, 2012, 120, 4571-82).

The integrity of the mucosal immune system appears to be critical for the rate of the HIV infection progression, because it is severely compromised during acute HIV infection. Beside the massive depletion of CD4+T cells, the frequency of IgA-producing B-cells in mucosal area drops dramatically, decreasing luminal secretory IgA concentrations involved in pathogens-immune exclusion protective pathway (described in both HIV and SIV infection; Alam et al., J. Virol., 2008, 82, 115-125; Muro-Cacho et al., J. Immunol., 1995, 154, 5555-5566; Douek et al., 2006, Nat. Immunol.,2006, 7, 235-239).

Transgenic mice expressing human chimeric IgA (EP patent 1 680 449) were used to ensure selection of a monoclonal IgA class antibody since a conventional immunization of wild type mice usually conduct to an IgG specific response. This mouse line was modified to express a constant human IgA heavy chain whereas constant light chain and variable genes remain from mouse origin. The C3G4-neutralizing antibody was induced by active immunization where the murine and not human repertoire has been involved in the antigen recognition. The possibility to generate the same response in human can only be evaluated in clinical trials.

Resistance to HIV infection is a rare phenomenon. Although several mechanisms may contribute including genetic factors (CCR5 d32 mutation, MHC allotype..) or immune factors such as specific IgA in vaginal secretions directed against the HIV-1 gp41, other factors may be involved.

The human and murine Ab response to many common pathogens is oligoclonal, with a restricted usage of Ig V-genes (Bos, N. A. et al., Infection and Immunity, 1996, 64, 616-623; Baxendale, H. E. & Goldblatt, D., Infection and Immunity 2006, 74, 1025-1031). In addition, in the mucosa exposed to a multitude of germs, these Abs are polyreactive and establish a natural immune homeostasis (Shimoda et al., Immunology, 1999, 97, 9-17).

Therefore, the inventors propose that the innate response to the genital tract microbiota (commensals or pathogens) could also play a role against HIV (or other pathogens) infection. The innate characteristic of the response is suggested by the oligoclonal profile of IgA found in the HEUW and the germinal feature of the Ab. The isolation of an Ab against *M. genitalium* cross-reactive with gp41 and neutralizing HIV-1 provides additional evidence. Whereas current thinking is that vaccine should induce hypermutated Ab in order to neutralize HIV-1 (Klein, F. et al., Cell, 2013,153, 126-38), the inventors propose that in mucosal site, main portal of entry of the virus, non affinity matured Ab elicited by specific commensals or pathogen, could neutralize and protect against HIV infection. HEUW may have a particular unique flora with native immune response that contributes to resistance to HIV-1 infection. Comparison of the microbiota in HEUW and women who later becomes infected will help to clarify this assumption.

The key for an effective vaccine strategy seems to be the identification of the priming antigen which elicits ancestor B lineage selection and proliferation for a future maturation all along its immune history. For some viral infections, the antigen that initially activates certain naive B-cell lineages and that stimulates the memory B-cells may not be identical.

The inventors propose that this population of naive B precursors may be recruited and primed by antigens, not only from past infections but from current natural commensal micro-organisms colonization. Immunization by *Mycoplasma genitalium* antigens induced cross-reactive anti-HIV antibodies and elicited a systemic and mucosal broadly neutralizing anti-HIV response. IgH variable segment of one of the neutralizing monoclonal antibodies from the present study reveals a highest homology with the murine germline ancestor IgVH5-12 (Figure 6). Interestingly, this particular murine segment shows a strong identity with the human unmutated IgVH1-2^{∗}2, ancestor of VRC01 broadly HIV neutralizing monoclonal antibody (bNab). Comparison of the IgVH regions highlights a conserved sequence covering 50 of the 98 amino acid residues, including five canonical HIV-contact residues, and particularly Arg⁷¹_{C3G4-VRC01}, which mimics the key interaction of Arg⁵⁹_{CD4} and Asp³⁶⁸_{gp120} (Scheid, J. F. et al., Science, 2011, 333, 1633-1637). Even if all critical residues are not conserved, the comparison of IgVH5-12 with the human IgVH1-2^{∗}2 (S18 and S19, Jardine and Schief , Science, may 2013, vol 340) suggested that the close conformational architecture of the two ancestors may be the key point of their ability to further neutralize HIV-1 infection, hIgVH1-2^{∗}2 targeting the gp120 whereas mIgVH5-12 targeting the gp41 on the surface of HIV-1 viral particles.

The inventors propose *M. genitalium* permease as a promising candidate to initiate a vaccine protocol mainly at the mucosal level based on its ability to prime naive B-cell clones, able to further recognize native HIV-antigens.

## Claims

1. A *Mycoplasma genitalium* permease antigen or a polynucleotide encoding said antigen in expressible form, for use in the prevention and/or treatment of HIV infection, wherein said permease antigen comprises the amino acid sequence of SEQ ID NO: 4 or an amino acid sequence which is at least 95 % identical to SEQ ID NO: 4 and which comprises an epitope cross-reactive with a HIV-gp41 ectodomain epitope, and wherein said antigen induces HIV cross-reactive and HIV neutralizing anti-permease antibodies.

2. The permease antigen for use according to claim 1, wherein said permease antigen consists of SEQ ID NO: 4.

3. The permease antigen for use according to claim 1, wherein said permease antigen comprises or consists of SEQ ID NO: 7.

4. The permease antigen for use according to any one of claims 1 to 3, wherein said permease epitope is cross-reactive with a HIV-gp41 ectodomain epitope from SEQ ID NO: 2.

5. The polynucleotide for use according to claim 1, wherein said polynucleotide comprises SEQ ID NO: 5 or 6.

6. The permease antigen or the polynucleotide encoding said antigen for use according to any one of claims 1 to 5, wherein the permease antigen or polynucleotide are contained in an immunogenic or vaccine composition comprising a pharmaceutically acceptable carrier and/or adjuvant.

7. The permease antigen or the polynucleotide encoding said antigen for use according 6, wherein the immunogenic or vaccine composition comprises an antibody directed to said permease antigen,

8. The permease antigen or the polynucleotide encoding said antigen for use according 7, wherein the immunogenic or vaccine composition comprises a humanized IgA antibody.

9. An isolated antibody or a functional fragment of said antibody comprising at least the antigen-binding site, wherein the antibody is directed against a *M. genitalium* permease antigen which comprises the amino acid sequence of SEQ ID NO: 4 or an amino acid sequence which is at least 95 % identical to SEQ ID NO: 4 and which comprises an epitope cross-reactive with a HIV-gp41 ectodomain epitope, and wherein said antibody is HIV cross-reactive and neutralizing.

10. The antibody of claim 9, which is a human chimeric monoclonal IgA.

11. The antibody of claim 10, which comprises an IgVH segment of SEQ ID NO: 8 and an IgVL segment of SEQ ID NO: 9.

## Patentansprüche

1. *Mycoplasma genitalium* Permease-Antigen oder ein Polynukleotid, das für das Antigen in exprimierbarer Form kodiert, zur Verwendung bei der Vorbeugung und/oder Behandlung einer HIV-Infektion, wobei das Permease-Antigen die Aminosäuresequenz von SEQ ID Nr: 4 umfasst oder eine Aminosäuresequenz, die mindestens 95 % zu SEQ ID Nr: 4 identisch ist und die ein Epitop umfasst, das mit einem Epitop der HIV-gp41 Ektodomäne kreuzreaktiv ist, und wobei das Antigen HIV-kreuzreaktive und HIV-neutralisierende anti-Permease-Antikörper induziert.

2. Permease-Antigen zur Verwendung nach Anspruch 1, wobei das Permease-Antigen aus SEQ ID Nr: 4 besteht.

3. Permease-Antigen zur Verwendung nach Anspruch 1, wobei das Permease-Antigen SEQ ID Nr: 7 umfasst oder aus dieser besteht.

4. Permease-Antigen zur Verwendung nach einem der Ansprüche 1 bis 3, wobei das Permease-Epitop mit einem Epitop der HIV-gp41 Ektodomäne aus SEQ ID Nr: 2 kreuzreaktiv ist.

5. Polynukleotid zur Verwendung nach Anspruch 1, wobei das Polynukleotid SEQ ID Nr: 5 oder 6 umfasst.

6. Permease-Antigen oder Polynukleotid, das für das Antigen kodiert, zur Verwendung nach einem der Ansprüche 1 bis 5, wobei das Permease-Antigen oder Polynukleotid in einer immunogenen oder Impfstoff-Zusammensetzung umfassend einen pharmazeutisch annehmbaren Träger und/oder Adjuvans enthalten sind.

7. Permease-Antigen oder Polynukleotid, das für das Antigen kodiert, zur Verwendung nach Anspruch 6, wobei die immunogene oder Impfstoff-Zusammensetzung einen Antikörper umfasst, der gegen das Permease-Antigen gerichtet ist.

8. Permease-Antigen oder das Polynukleotid, das für das Antigen kodiert zur Verwendung nach Anspruch 7, wobei die immunogene oder Impfstoff-Zusammensetzung einen humanisierten IgA-Antikörper umfasst.

9. Isolierter Antikörper oder ein funktionelles Fragment des Antikörpers umfassend mindestens die Antigenbindestelle, wobei der Antikörper gegen ein *M*. *genitalium* Permease-Antigen gerichtet ist, das die Aminosäuresequenz von SEQ ID Nr: 4 umfasst oder eine Aminosäuresequenz, die mindestens 95 % zu SEQ ID Nr: 4 identisch ist und die ein Epitop umfasst, das mit einem Epitop der HIV-gp41 Ektodomäne kreuzreaktiv ist, und wobei der Antikörper HIV-kreuzreaktiv und - neutralisierend ist.

10. Antikörper nach Anspruch 9, der ein humaner chimärer monoklonaler IgA ist.

11. Antikörper nach Anspruch 10, der ein IgVH-Segment von SEQ ID Nr: 8 und ein IgVL-Segment von SEQ ID Nr: 9 umfasst.

## Revendications

1. Antigène de perméase de *Mycoplasma genitalium* ou polynucléotide codant pour ledit antigène sous une forme exprimable, pour son utilisation dans la prévention et/ou le traitement d'une infection par le VIH, dans lequel ledit antigène de perméase comprend la séquence d'acides aminés de SEQ ID NO : 4 ou une séquence d'acides aminés qui présente une identité d'au moins 95 % avec SEQ ID NO : 4 et qui comprend un épitope qui présente une réaction croisée avec un épitope d'ectodomaine de la gp41 du VIH, et dans lequel ledit antigène induit des anticorps anti-perméase neutralisant le VIH et présentant une réaction croisée avec le VIH.

2. Antigène de perméase pour son utilisation selon la revendication 1, dans lequel ledit antigène de perméase consiste en SEQ ID NO : 4.

3. Antigène de perméase pour son utilisation selon la revendication 1, dans lequel ledit antigène de perméase comprend ou consiste en SEQ ID NO : 7.

4. Antigène de perméase pour son utilisation selon l'une quelconque des revendications 1 à 3, dans lequel ledit épitope de la perméase présente une réaction croisée avec un épitope d'ectodomaine de la gp41 du VIH de SEQ ID NO : 2.

5. Polynucléotide pour son utilisation selon la revendication 1, dans lequel ledit polynucléotide comprend SEQ ID NO : 5 ou 6.

6. Antigène de perméase ou polynucléotide codant pour ledit antigène pour son utilisation selon l'une quelconque des revendications 1 à 5, dans lequel l'antigène de perméase ou le polynucléotide sont contenus dans une composition immunogène ou vaccinale comprenant un véhicule et/ou un adjuvant pharmaceutiquement acceptables.

7. Antigène de perméase ou polynucléotide codant pour ledit antigène pour son utilisation selon la revendication 6, dans lequel la composition immunogène ou vaccinale comprend un anticorps dirigé contre ledit antigène de perméase.

8. Antigène de perméase ou polynucléotide codant pour ledit antigène pour son utilisation selon la revendication 7, dans lequel la composition immunogène ou vaccinale comprend un anticorps IgA humanisé.

9. Anticorps isolé ou fragment fonctionnel dudit anticorps comprenant au moins un site de liaison à un antigène, dans lequel ledit anticorps est dirigé contre un antigène de perméase de *M. genitalium* qui comprend la séquence d'acides aminés de SEQ ID NO : 4 ou une séquence d'acides aminés qui présente une identité d'au moins 95 % avec SEQ ID NO : 4 et qui comprend un épitope présentant une réaction croisée avec un épitope d'ectodomaine de la gp41 du VIH, et dans lequel ledit anticorps présente une réaction croisée avec le VIH et le neutralise.

10. Anticorps selon la revendication 9, qui est une IgA monoclonale chimèrique humaine.

11. Anticorps selon la revendication 10, qui comprend un segment VH d'Ig de SEQ ID NO : 8 et un segment VL d'Ig de SEQ ID NO : 9.
